# EUROPEAN PATENT SPECIFICATION

(11) **EP 3 675 795 B1**
(45) Date of publication and mention of the grant of the patent: **18.08.2021**
(21) Application number: 18849703.6
(22) Date of filing: 27.08.2018
(51) Int. Cl.: A61H 23/00, A61H 23/02, A61N 1/00, A61N 1/04, A61N 1/36, A61H 9/00

(54) **SYSTEMS FOR CONTROLLING THE EFFECTS OF TREMORS**
SYSTEME ZUR STEUERUNG DER AUSWIRKUNGEN VON TREMORES
SYSTÈMES DESTINÉS AU CONTRÔLE DES EFFETS DES TREMBLEMENTS

(30) Priority: 01.09.2017 US 201762553683 P; 01.02.2018 US 201862624896 P; 23.05.2018 US 201862675372 P
(43) Date of publication of application: 08.07.2020
(73) Proprietor: Adventus Ventures, LLC, Irvine, California 92604 (US)
(72) Inventor: MOADDEB, Shahram, Irvine, California 92604 (US); WOODS, Carla Mann, Beverly Hills, California 90210 (US); SAMA, Rinda, Laguna Niguel, California 92677 (US); ABDEEN, Faizal, Mission Viejo, California 92692 (US)
(74) Representative: Lecomte & Partners
(86) International application number: PCT/US2018/048118
(87) International publication number: WO 2019/046180

(56) References cited:
- EP-A1- 2 801 389
- US-A- 5 131 401
- US-A1- 2004 024 312
- US-A1- 2004 082 979
- US-A1- 2005 080 463
- US-A1- 2010 249 677
- US-A1- 2012 245 483
- US-A1- 2013 041 296
- US-A1- 2015 174 391
- US-A1- 2015 297 909
- US-A1- 2017 120 060
- US-A1- 2017 165 486

## Description

### BACKGROUND OF THE INVENTION

Embodiments of the present invention relate to systems and methods for controlling the effects of tremors.
The document US 2017/0165486 A1 discloses a neuro-stimulation system used for rehabilitation of individuals who suffer loss of sensorimotor function following stroke. The system applies neuro-stimulation to sensory cells of body parts during movement of the body parts to induce neuroplastic changes.

### SUMMARY OF THE INVENTION

In a first embodiment of the present disclosure, a system for treatment of involuntary muscle contraction includes a wearable interface having an internal contact surface, the wearable interface configured to at least partially encircle a first portion of a limb of a subject, and an energy applicator carried by the wearable interface and configured to apply energy of two or more types to the limb of the subject. In some embodiments, the system for treatment of involuntary muscle contraction further comprises a control unit configured to control the operation of the energy applicator. The invention is defined in claim 1, preferred embodiments are disclosed in the dependent claims.

### BRIEF DESCRIPTION OF THE DRAWINGS

FIG. 1 is a perspective view of a wearable tremor control system, according to an embodiment of the present disclosure.
FIG 2 is a perspective view of the wearable tremor control system of FIG. 1 in a fastened, unexpanded condition.
FIG. 3 is a perspective view of the wearable tremor control system of FIG. 1 in a fastened, partially expanded condition.
FIG. 4 is a perspective view of the wearable tremor control system of FIG. 1 in a fastened, substantially expanded condition.
FIG. 5 is a perspective view of the wearable tremor control system of FIG. 1 in use on the wrist of a user.
FIG. 6 is a perspective view of the wearable tremor control system of FIG. 1 during the detection of a tremor.
FIG. 7 is a perspective view of the wearable tremor control system of FIG. 1 during activation in response to a detected tremor.
FIG. 8 is a perspective view of a wearable tremor control system, according to an embodiment of the present disclosure.
FIG. 9 is partially cutaway perspective view of the wearable tremor control system of FIG. 8 in a first state.
FIG. 10 is partially cutaway perspective view of the wearable tremor control system of FIG. 8 in a second state.
FIG. 11 is partially cutaway perspective view of the wearable tremor control system of FIG. 8 in a third state.
FIG. 12 is a cross-sectional view of the wearable tremor control system of FIG. 8 in a first state.
FIG. 13 is a cross-sectional view of the wearable tremor control system of FIG. 8 in a second state.
FIG. 14 is a cross-sectional view of the wearable tremor control system of FIG. 8 in a third state.
FIG. 15 is a magnified view of the wearable tremor control system of FIG. 12.
FIG. 16 is a plan view of a user interface of the wearable tremor control system of FIG. 8.
FIG. 17 is a perspective view of the wearable tremor control system of FIG. 8 in use on the ankle of a user.
FIG. 18 is cross-sectional view of a wearable tremor control system according to an embodiment of the present disclosure.
FIG. 19 is a perspective view of a wearable tremor control system, according to an embodiment of the present disclosure.
FIG. 20 is a perspective view of a wearable tremor control system, according to an embodiment of the present disclosure.
FIG. 21 is a perspective view of the wearable tremor control system of FIG. 20 in a decoupled state.
FIG. 22 is a perspective view of a wearable tremor control system in use on the wrist of a user, according to an embodiment of the present disclosure.
FIG. 23 is a cross-sectional view of the wearable tremor control system of FIG. 22 in a first state.
FIG. 24 is a cross-sectional view of the wearable tremor control system of FIG. 22 in a second state.
FIG. 25 is a cross-sectional view of the wearable tremor control system of FIG. 22 in a third state.
FIG. 26 is a plan view of key components of the wearable tremor control system of FIG. 22.
FIG. 27 is a cross-sectional view of the wearable tremor control system of FIG. 26, taken along line 27.
FIG. 28 is a perspective view of a wearable tremor control system, according to an embodiment of the present disclosure.
FIG. 29 is an exploded view of the wearable tremor control system of FIG. 28.
FIG. 30 is an exploded view of the wearable tremor control system of FIG. 28.

### DETAILED DESCRIPTION OF THE ILLUSTRATED EMBODIMENTS

A tremor is an involuntary, muscle contraction leading to shaking or cyclic movement in one or more parts of the body. The muscle contraction often follows a rhythmic pattern. It is common for tremor to affect the hand or wrist area of a sufferer, but arms, legs, head, torso, and even vocal cords may also be affected. Tremor may be intermittent or in some cases may be constant or permanent. In some cases, tremor accompanies or is accompanied by one or more other disorders. The effects of tremor can be partially or severely disabling, and are often the cause of embarrassment. Some forms of tremor include essential tremor, restless leg syndrome (RLS), Parkinson's tremor, dystonic tremor, cerebellar tremor, resting tremor, action tremor, psychogenic tremor (related to psychological disorders), enhanced physiologic tremor, or orthostatic tremor. In some cases, surgery may be performed in order to to treat tremor, such as deep brain stimulation (DBS) and thalamotomy. Though improvement can be seen after these procedures, the procedures may also be the cause pf subsequent speech or balance problems in the patient.

Some pharmacologic means currently used to treat types of tremor include anti-seizure medications, including topiramate or gabapentin, beta blockers, such as propranolol, atenolol, metoprolol, nadolol, and sotalol, benzodiazepine tranquilizers, such as alprazolam or clonazepam, Parkinson's disease medications, such as levodopa or carbidopa, and in some cases, medications such as botulinum toxin (BTX). All of these medications may have certain side effects that are undesirable for the particular sufferer of tremor.

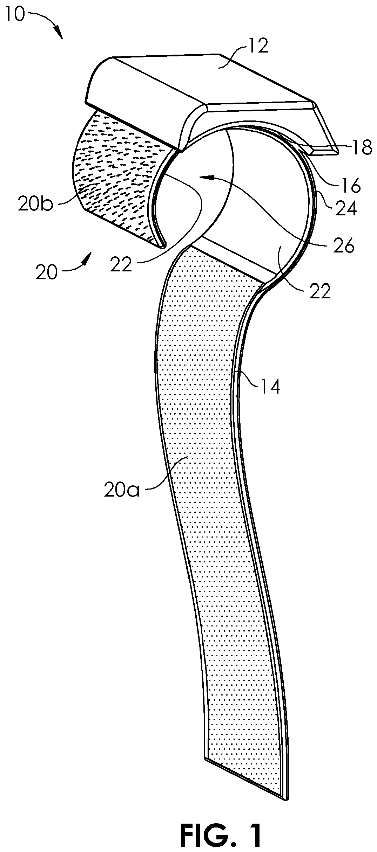

Essential tremor (or familial tremor) is a shaking that usually occurs in one or both arms, wrists, or hands of a sufferer. However, the head or voice of the sufferer may also be affected. FIG. 1 illustrates a wearable tremor control system 10 configured for placement on the wrist of a patient. The wearable tremor control system 10 comprises a housing 12, and a band 14 coupled to an underside 16 of the housing 12. The wearable tremor control system 10 is shown in FIG. 1 in an unfastened condition. The band 14 is secured to the underside 16 of the housing 12 by epoxy or adhesive 18. In other embodiments, the housing 12 may be overmolded or insert molded in conjunction with the band 14. In other embodiments, the band 14 may be secured by fasteners, sewing, fusing, or may slide through slits or elongate spaces in the housing 12. The band 14 may even be configured to remain slidable (e.g., longitudinally along its own axis) within the grooves or slots, in order to adjust the position of the band 14 in relation to the housing 12. The band 14 is configured to wrap around the wrist of a user/patient and secure to itself by use of a hook and loop (Velcro®-type) system 20. The loop surface 20a on an interior of a portion of the band 14 secures to the hook surface 20b on an exterior portion of the band 14. An inflatable cuff 22 extends around a circumferential path 24 encircling an interior 26 of the band 14. In some embodiments, the band 14 may be configured to be worn like a watch or a bracelet, and may be configured to partially or fully encircle a limb (arm, leg) at a particular portion (wrist, ankle, etc.). The hook and loop system 20 may be replaced in alternative embodiments by a button closure, a snap closure, loop closure, an adhesive closure, or a magnetic closure.

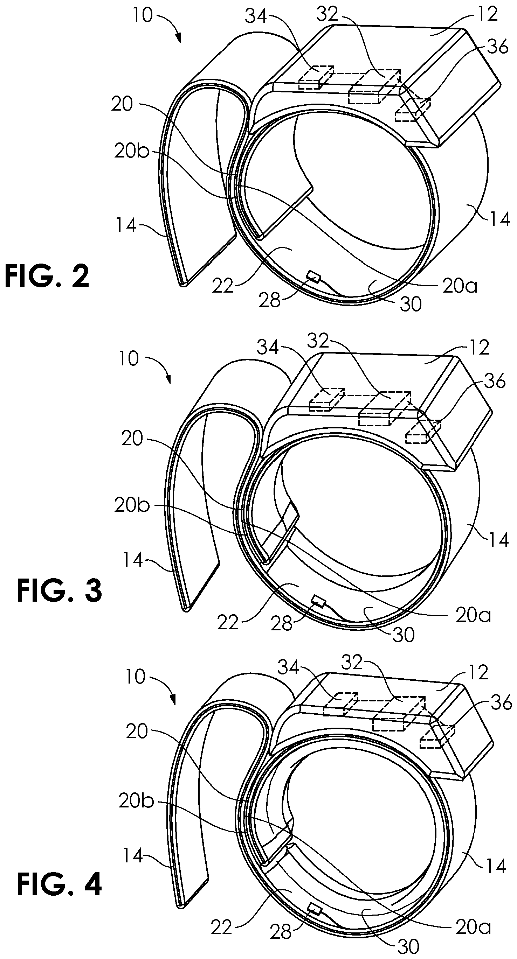

FIG. 2 illustrates the wearable tremor control system 10 in a fastened condition, with the loop surface 20a secured to the hook surface 20b. No arm of a user is shown in FIGS. 2-4 in order to better show the activity of the inflatable cuff 22. A sensor 28 is carried on an interior face 30 of the band 14, and is configured to sense disturbances caused by activity of the muscle. In some embodiments, the sensor 28 may comprise an ultrasound transducer. In some embodiments, the sensor 28 may comprise an accelerometer configured to measure acceleration (e.g, of the limb during tremor), and may further comprise a piezoelectric accelerometer, a piezoresistive accelerometer, or a capacitive accelerometer. In some embodiments, the sensor 28 may comprise a gyroscope. The gyroscope may be configured to measure angular velocity (e.g., of the limb during tremor). In some embodiments, the sensor 28 may comprise an electrogoniometer configured to provide a signal related to angle (e.g., elbow joint angle) over time. In some embodiments, the sensor 28 may comprise a force gauge or strain gauge. In some embodiments, the sensor 28 may comprise an electromyography (EMG) sensor. In some embodiments, the sensor 28 may comprise two or more different types of sensors, such as those previously described. Multi-modal sensing is thus possible. A controller 32 within the housing 12 is configured to receive signals from the sensor 28. The controller 32 may comprise a microcontroller, and is electrically coupled to the sensor 28. The controller 32 is also electrically coupled to a transceiver 34, the transceiver configured to communicate wirelessly to a cellular phone, smart phone, or other personal communication device. The personal communication device may include a chip (e.g, integrated circuit) implanted in a user's body, or a chip carried on a portion of the user's body or clothing. The transceiver 34 may in some embodiments comprise a wifi antenna. An actuator 36, coupled to the controller 32 is configured to receive signals from the controller 32 to cause the inflatable cuff 22 to expand. The actuator 36 may comprise a number of different mechanical, hydraulic, or pneumatic apparatus to cause the cuff 22 to inflate, or otherwise expand. The inflatable cuff 22 is shown in FIG. 2 in a substantially unexpanded condition. The inflatable cuff 22 in FIG. 3 is shown in a partially expanded condition. The inflatable cuff 22 in FIG. 4 is shown in a substantially expanded condition. In the partially expanded condition of FIG. 3, the inflatable cuff 22 may begin to apply a pressure on the limb or may increase a pressure applied on the limb. In the substantially expanded condition of FIG. 4, the inflatable cuff 22 may apply a sufficient pressure on the limb to create an effect or to optimize an effect. Though a power unit is not shown in FIGS. 2-4, a battery may be included within the housing, to power the electrical components. The battery may be rechargeable, or removable, or disposable. An alternative powering means to a battery may be used, such as an inductively coupling power circuit or a wirelessly chargeable capacitor.

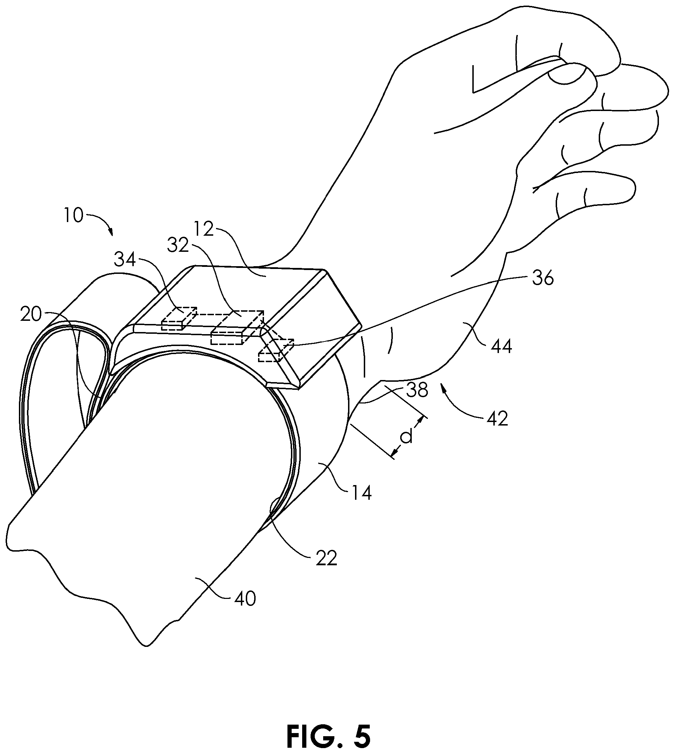

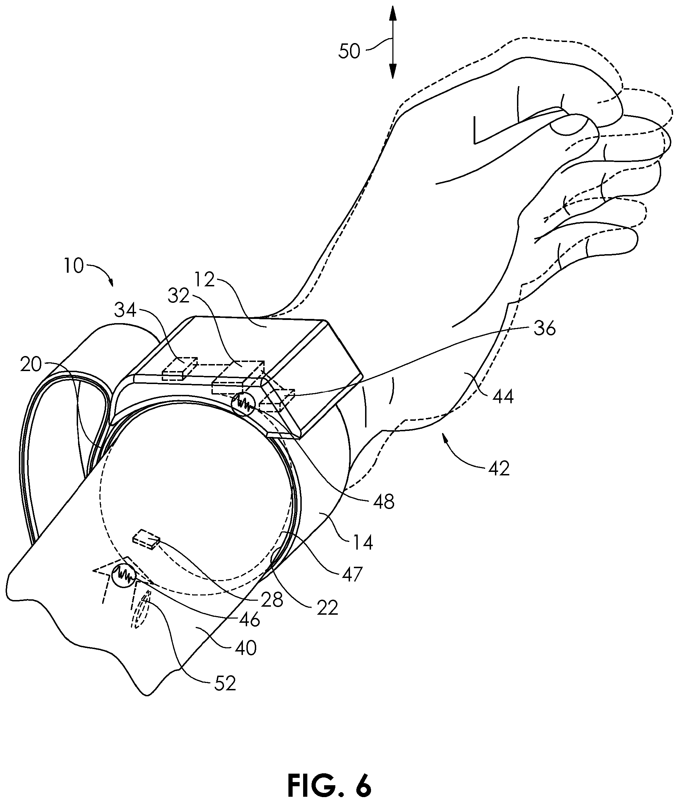

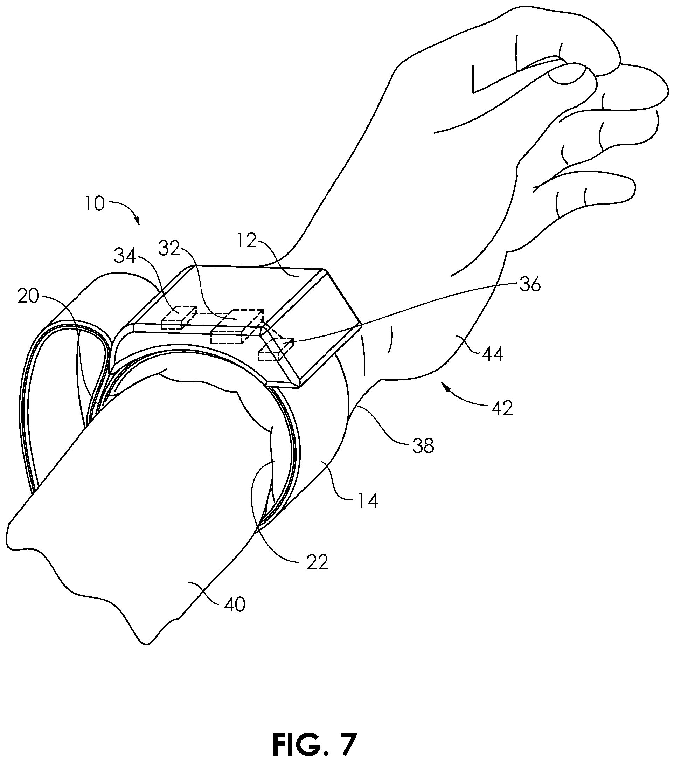

In FIG. 5, the wearable tremor control system 10 is shown in use, in place on the wrist 38 of the arm 40 of a user 42. The band 14 may be secured immediately adjacent the hand 44 of the user 42, or may be attached around the wrist 38 (or other portion of the arm 40) a distance d away from the hand 44, for example 0.5 cm, 1 cm, 2 cm, 5 cm, 10 cm, or 15 cm, or any distance between 0 cm and 15 cm. The band 14 may alternatively be secured around a portion of the upper arm (not shown). Turning to FIG.6, the user 42 experiences a tremor (bi-directional displacement arrow 50) caused at least partially by involuntary activity 46 of one or more muscles 52 in the vicinity of the sensor 28. The sensor 28 outputs a signal 48 proportional to the activity 46, and the signal 48 is received by the controller 32 (e.g., via a conductor 47). In some embodiments, wherein the sensor 28 comprises a piezoelectric sensor, the sensor 28 may output a signal 48 of between about 0.1 milliVolt and about 10 Volts when responding to displacement caused by the shaking of a limb. In FIG. 7, the controller 32 commands the actuator 36 to expand the inflatable cuff 22 against the wrist 38 of the user 42. The controller 32 may command the actuator 36 to expand the inflatable cuff 22 at least partially based upon data received via the signal 48 from the sensor 28. For example, if the signal 48 is above a particular threshold amplitude or if the signal 48 last longer than a particular threshold duration, the controller 32 may command the actuator 36 the expand the inflatable cuff 22. The expansion pressure of the inflatable cuff 22 may even be based on an algorithm including a parameter of the signal 48.

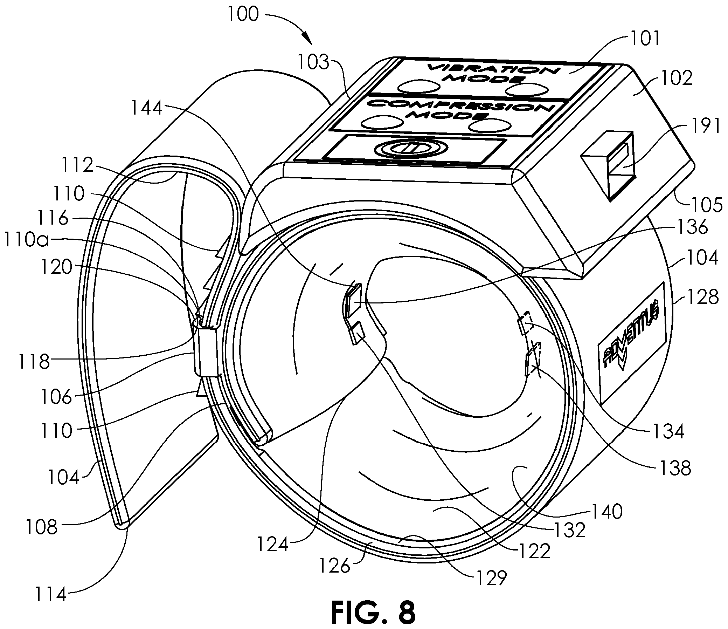

FIG. 8 illustrates a wearable tremor control system 100 configured for placement on the wrist 38 of a patient. The wearable tremor control system 100 comprises a housing 102, and a band 104 coupled to an underside 105 of the housing 102. The wearable tremor control system 100 is shown in FIG. 8 in a fastened condition, though without the arm 40 of the user 42 visible, in order to better show features of the wearable tremor control system 100. A loop 106 is secured to a first portion 108 of the band 104 and a series of rubber wedges 110 are carried by a second portion 112 of the band 104. The wedges 110 may be made from any compressible or semi-compressible material, and may be bonded or otherwise secured to the band 104, or may be molded directly on the band 104, or may be an inherent or integral part of the band 104. To attach the wearable tremor control system 100 to the user's wrist 38, the user 42 (or a person aiding the user 42) slips a first end 114 of the band 104 through an opening 116 of the loop 106 and, while applying traction on the first end 114, pulls one or more of the wedges 110 through the opening 116 of the loop 106, until the band 104 is at a comfortable tightness around the user's wrist 38. A flat edge 118 of one of the wedges 110a, abuts a flat edge 120 of the loop 106, locking the band 104 in place. To remove the band 104, the user 42 may force the band 104 in the opposite direction, deforming the wedges 110 as they are pulled through the opening 116 in the loop 106. Alternatively, a hook and loop 20, like that of the wearable tremor control system 10 of FIG. 1 may be used. The band 104 may be provided in a number of different models or sizes, to best optimize placement on a particularly-sized patient (e.g., small, medium, large or pediatric, adult). The wearable tremor control system 100 also includes a user interface 101 carried on a visible surface 103 of the housing 102.

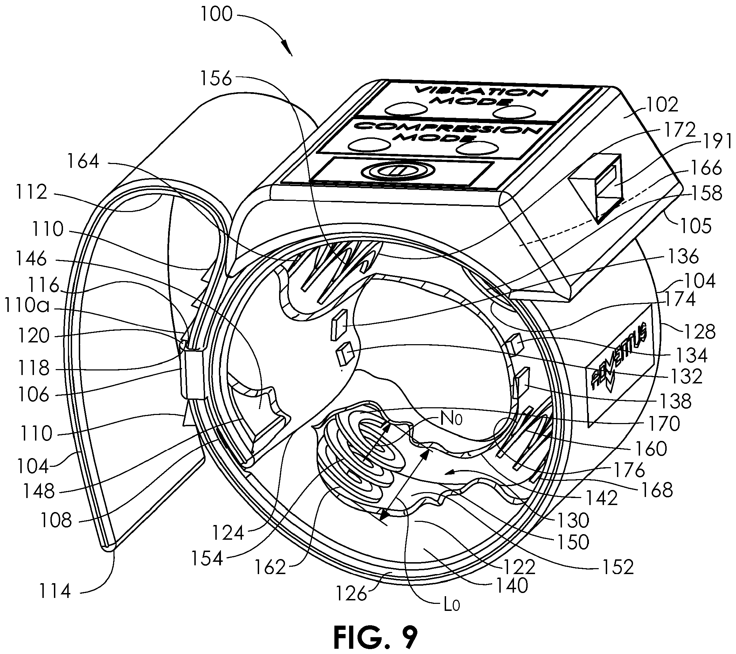

The wearable tremor control system 100 includes an outer cuff 122 extending circumferentially within the band 104 between a second end 124 of the band 104 and the first portion 108. The outer cuff 122 is secured to the band 104 along a first edge 126 and a second edge 128, each running circumferentially around an internal periphery 129 of the band 104. The outer cuff 122 may be secured to the band 104 at the first and second edges 126, 128 by adhesive, epoxy, or hotmelt, or may be sewn, molded, stapled, or secured with other fastening means. The outer cuff 122, as named, represents an outer layer, though it is an inner portion of a circle when attached. As shown in FIG. 9, the outer cuff 122 is configured to have an interior space 130 in which other dynamic components of the wearable tremor control system 100 are able to move.

The outer cuff 122 carries a pair of sensing elements 132, 134 (e.g., sensors or transducers) and a pair of vibration elements 136, 138. In some embodiments, the sensing elements 132, 134 may comprise piezo crystals, and may be configured to vibrate at between about 40 Hz and about 500 Hz, or between 50 Hz and about 450 Hz, or between about 60 Hz and about 400 Hz, or between about 100 Hz and about 350 Hz. The sensing elements 132, 134 are configured to sense physiological signals from a user's limb related to muscle contraction, including movement, which is sensed as a displacement. Physiological signals that are indicative of tremor tend to include a repetitive wave form that a motion sensor (sensing elements 132, 134) is capable of measuring. The vibration elements 136, 138 may comprise piezoelectric crystals, and may be configured to vibrate between about one Hz and about 30 Hz, or between about two Hz and about 15 Hz, or between about three Hz and about 10 Hz. Frequencies between about one Hz and about 30 Hz can be very effective at dampening the shaking of a patient's limb (arm, wrist, etc.), and thus the vibration elements 136, 138, when constructed of an appropriate material and having an appropriate thickness to vibrate at one or more frequencies in the 1-30 Hz range, may be configured to abate or completely stop the shaking caused by one or more forms of tremor. The piezoelectric crystals may comprise quartz, artificial quartz, or PZT (lead zirconate titanate) ceramics. In other cases, the vibration elements 136, 138 may comprise piezo crystals, and may be configured to vibrate at ultrasound frequencies of between about 15 kHz and about 1 MHz, or about 20 kHz and about 700 kHz, or between about 20 kHz and 500 kHz, or between about 25 kHz and about 500 kHz, or between about 30 kHz and about 500 kHz, or between about 30 kHz and about 200 kHz, or between about 20 kHz and about 200 kHz, or between about 100 kHz and about 300 kHz. Frequencies between about 20 kHz and about 700 kHz can be very effective at stimulating nerves, such as the median nerve in the arm. Thus, the vibration elements 136, 138, when constructed of an appropriate material and having an appropriate thickness to vibrate at one or more frequencies in the 15 kHz to 1 MHz range, or more specifically the 20-700 kHz range, may be configured to stimulate the median nerve via vibration. The applied vibration to the median nerve will be sensed in the brain of the user, which will alter limb shaking accordingly as part of a physiological feedback loop. The brain is thus "tricked" into playing a more involved interventional role. In some cases, the applied vibration may reduce activity of the thalamus, particularly in its contribution to control and coordination of muscle movement. In some embodiments, one vibration element 136 may be configured to vibrate within one of the lower frequency ranges (e.g., 1-30 Hz, 2-15 Hz, 3-10 Hz) while the other vibration element 138 may be configured to vibrate at one of the higher (ultrasound) frequency ranges (e.g., 20-700 kHz, 25-500 kHz, 30-200 kHz), in order to induce both types of effect. In other embodiments two or more vibration elements 136, 138 may be configured to vibrate within one of the lower frequency ranges (e.g., 1-30 Hz, 2-15 Hz, 3-10 Hz) while two or more additional vibration elements 136, 138 (not shown) may be configured to vibrate at one of the higher (ultrasound) frequency ranges (e.g., 20-700 kHz, 25-500 kHz, 30-200 kHz). In some embodiments, one or more vibration elements 136, 138 may be configured to vibrate at multiple frequencies, for example a fundamental frequency (or first harmonic) and a second harmonic. The first harmonic, for example, in a particular embodiment may be 10 Hz, and a second harmonic may be 20 Hz. In another embodiment, first harmonic may be 150 kHz and the second harmonic may be 300 kHz. In other embodiments, a third harmonic, or even fourth, fifth, or higher harmonics may be used, as described by the harmonic series. One particular treatment protocol may comprise a first period of activation of the vibration elements 136, 138 which is initiated immediately after the sensing elements 132, 134 detect a tremor, or more specifically, after signals are received from one or more of the sensing elements 132, 134 that are in a range that is indicative to active tremor. This first period of activation may be followed by pressurization of an inflatable inner cuff 146 within the outer cuff 122, as will be described in more detail. In relation to FIGS. 1-7, a further embodiment of the wearable tremor control system 10 may add vibration elements 136, 138. A particular treatment protocol associated with this alternative embodiment of the wearable tremor control system 10 may comprise a first period of activation of the additional vibration elements 136, 138 which is initiated immediately after the sensor 28 detects a tremor or sensed signals related thereto. This first period of activation may be followed by an increase in pressurization of the inflatable cuff 22.

In some embodiments, the sensing elements 132, 134 and vibration elements 136, 138 may be replaced by multi-purpose elements which are configured to perform both the sensing function of the sensing elements 132, 134 and the energy application function of the vibration elements 136, 138. In some embodiments, one or more of the sensing elements 132, 134 and/or vibration elements 136, 138 may include a mechanical displacement amplifier to improve energy transfer to (or from) a wearer/patient.

One or more of the sensing elements 132, 134 or vibration elements 136, 138 may be carried on an outer surface 140 of the outer cuff 122, or may be carried on an inner surface 142 (FIG. 9) of the outer cuff 122, or a combination thereof. The outer cuff 122 is configured to maintain the sensing elements 132, 134 and vibration elements 136, 138 in proximity to the wrist 38 of the user 42 (or other portion of any limb upon which the band 104 has been attached). It may be desired to cover the wrist 38 with an acoustic coupling gel, or other acoustic coupling media, for optimal acoustic coupling between skin of the user 42 and the sensing elements 132, 134 or vibration elements 136, 138. The sensing elements 132, 134 and vibration elements 136, 138 can be secured to the outer surface 140 and/or inner surface 142 of the outer cuff 122 by an epoxy or adhesive 144 that has appropriate transition acoustic impedance properties.

Within the interior space 130 of the outer cuff 122, an inflatable inner cuff 146 (FIG. 9) is secured to the band 104 along a first edge 148 and a second edge 150, each running circumferentially around an internal periphery of the band 104, within the outer cuff 122. The inner cuff 146 may be secured to the band 104 at the first and second edges 148, 150 by adhesive, epoxy, or hotmelt, or may be sewn, stapled, or secured with other fastening means. The inner cuff 146 includes a surface 152 upon which are secured four compression springs 154, 156, 158, 160. First ends 162, 164, 166, 168 of the compression springs 154, 156, 158, 160 may be secured to the surface 152 with an epoxy or adhesive. In alternate embodiments, the inner cuff 146 comprises a composite structure including a woven layer which provides the surface 152, wherein the compression springs 154, 156, 158, 160 are woven, tied, or otherwise combined with the woven layer. Second ends 170, 172, 174, 176 of the compression springs 154, 156, 158, 160 are configured to abut the inner surface 142 of the outer cuff 122.

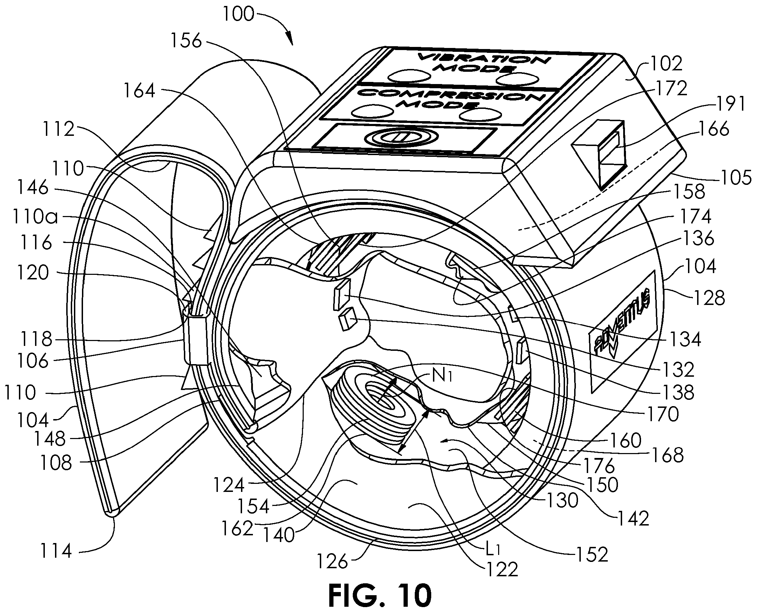

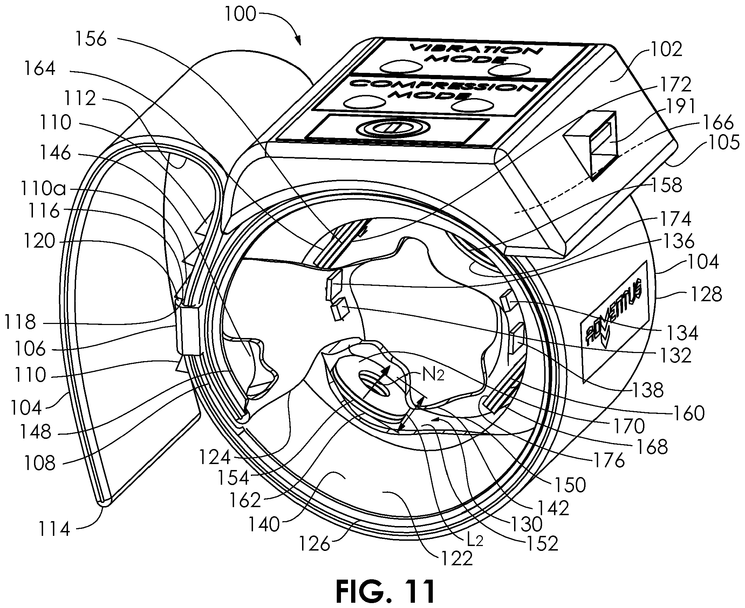

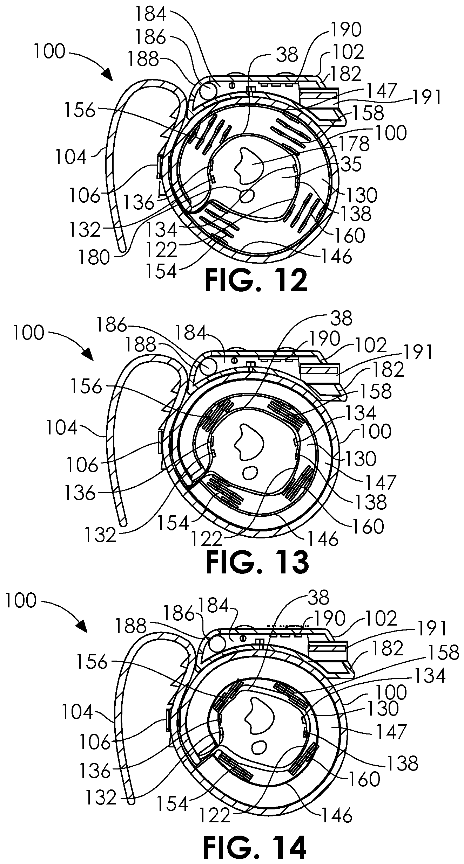

In FIG. 9, the inner cuff 146 is in a first, substantially uninflated, state, and the second ends 170, 172, 174, 176 of the compression springs 154, 156, 158, 160 apply little or no radially-directed normal force N₀ against the outer cuff 122 (e.g., at the inner surface 142). The compression springs 154, 156, 158, 160 are thus not substantially compressed or not compressed at all, and thus maintain their unstressed lengths L₀. Note that in some embodiments, each compression spring 154, 156, 158, 160 may have a different length or orientation, or even a different material than the other compression springs 154, 156, 158, 160, and thus each of their lengths and/or resulting normal forces may differ from each other. In FIG. 10, the inner cuff 146 is in a second, semi-inflated or partially inflated, state. From the effect of this inflation, the surface 152 of the inner cuff 146 expands inwardly, to a decreased diameter, forcing the second ends 170, 172, 174, 176 of the compression springs 154, 156, 158, 160 into the inner surface 142 of the outer cuff 122 with an increased normal force N₁. The inner cuff 146 has an interior space 147 (FIGS. 12-15) whose volume increases as the inner cuff 146 is inflated (e.g., as the interior pressure is increased). The compression springs 154, 156, 158, 160 are in turn compressed to a length L₁ by this normal force N₁. Thus, the length L₁ and normal force N₁ shown in FIG. 10 represent equilibrium values as the surface 152 has compressed the compression springs 154, 156, 158, 160 against the inner surface 142 of the outer cuff 122. In FIG. 11, the inner cuff 146 is in a third, substantially inflated, state. From the effect of this inflation, the surface 152 of the inner cuff 146 expands further inwardly, to a further decreased diameter, forcing the second ends 170, 172, 174, 176 of the compression springs 154, 156, 158, 160 even more into the inner surface 142 of the outer cuff 122 with a further increased normal force N₂. The compression springs 154, 156, 158, 160 are in turn further compressed to a length L₂ by this normal force N₂. Thus, N₂ is greater than N₁ and N₁ is greater than N₀. Furthermore, L₂ is less than L₁ and L₁ is less than L₀. Because the band 104 of the wearable tremor control system 100 is secured around the user's wrist 38, the normal forces N₀, N₁, N₂ are each applied against the wrist 38, each a different location, for example, at four equally-spaced, or equally distributed quadrants. The equal distribution may in some embodiments be different than equal spacing, for example, because a non-circular cross-section of a human wrist may dictate different spacing (such as the four vertices of a rectangle) to distribute forces on the wrist. The compression springs 154, 156, 158, 160 typically have a particular spring coefficient k by which the normal force Nᵢ is proportional to the compressed length Lᵢ by the formula Nᵢ = -k × Lᵢ. The outer cuff 122 is between the second ends 170, 172, 174, 176 of the compression springs 154, 156, 158, 160 and the wrist 38, and serves as a buffer layer to protect the wrist 38 from any lacerations, abrasions, or contusions, while still allowing the normal forces N₀, N₁, N₂ to be applied to the wrist 38. An increase of the inflation of the inner cuff 146 causes the increase in the applied normal forces (No increasing to N₁, or to N₂) applied against the wrist 38. In some embodiments, a washer or disc may be added at the second ends 170, 172, 174, 176 of the compression springs 154, 156, 158, 160 to create an annular or circular compression surface. The washer or disc may comprise a hard material, such as stainless steel or other metals or a relatively rigid polymeric material such as nylon (polyamide), polyimide, or PEEK. However, as shown in FIG. 11, the compression springs 154, 156, 158, 160 in their substantially compressed states can apply the effect of a substantially annular compression surface to the wrist 38, which can be evened out by the effect of the intermediate outer cuff 122.

FIG. 12 illustrates the wearable tremor control system 100 in use on a wrist 38 of a user 42 with the inner cuff 146 in a first, substantially uninflated, state. Radius 178 and ulna 180 bones are shown in the cross-section of the wrist 38, as the cross-section is taken through a portion of the wearable tremor control system 100 that is proximal to the carpal bones. Muscle 35 is also shown surrounding the radius 178 and ulna 180. FIG. 13 illustrates the wearable tremor control system 100 in use on a wrist 38 of a user 42 with the inner cuff 146 in a second, semi-inflated or partially inflated, state. FIG. 14 illustrates the wearable tremor control system 100 in use on a wrist 38 of a user 42 with the inner cuff 146 in a third, substantially inflated, state. The increase in the volume of the interior space 147 of the inner cuff 146 is visible, progressing from FIG. 12 to FIG. 13 and from FIG. 13 to FIG. 14. The resultant increased compression of the compression springs 154, 156, 158, 160 is also visible.

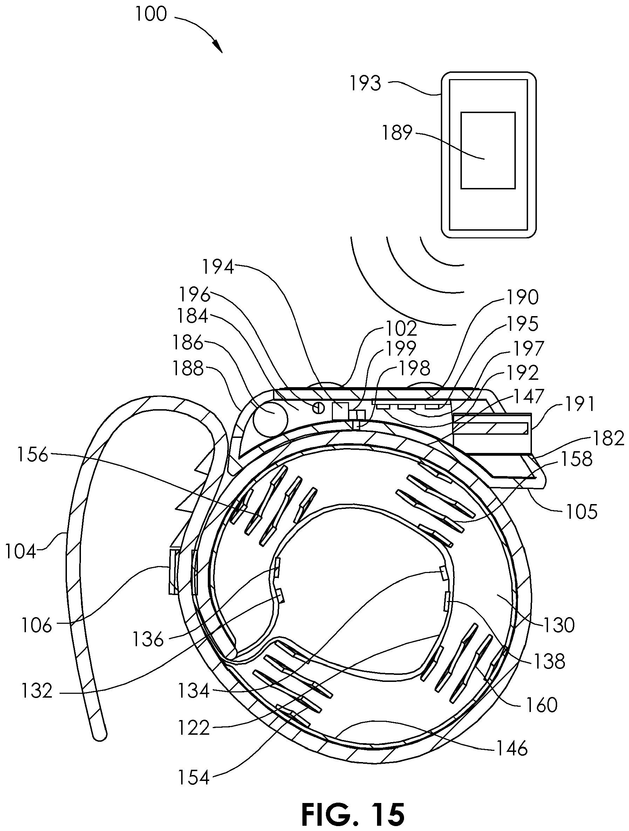

Turning to FIG. 15, the wearable tremor control system 100 is shown in further detail, but without the wrist 38 visible. The housing 102 of the wearable tremor control system 100 comprises a wall 182 and an internal cavity 184. A battery 186 is held within the internal cavity 184 and covered with a removable batter cover 188. The battery 186 is configured to power a circuit board 190 of the wearable tremor control system 100. The circuit board 190 includes a controller 192 which is configured to control a pneumatic pump 194. The pneumatic pump 194 is configured to pump air that enters the internal cavity 184 through an intake/outlet hole 196 and force the air through an inflation tube 198 that passes from the internal cavity 184 through the wall 182 and into the interior space 147 of the inner cuff 146. A valve 199 is configured to be closable to hold a constant pressure within the interior space 147 of the inner cuff 146 when it is inflated. The valve 199 may be an electromagnetically operated valve (e.g., micro solenoid), or may be a mechanical valve (e.g., pinch valve). The circuit board 190 further comprises a memory unit 197 which is configured to store data, such as patient data, calibration data, treatment programs, treatment data (e.g., reduction or increase in amplitude, intensity and/or prevalence of tremor), and measurement algorithms. The circuit board 190 also includes a transceiver 195 which is configured to communicate with an external device 193, such as a smart phone, pad, personal computer, or other device capable of communication. The external device 193 may include an application (App) 189 that allows the users to control and modify the operation of the wearable tremor control system 100. The application may comprise a computer program embodied in a non-transitory computer readable medium, that when executing on one or more computers (e.g., the external device 193) provides one or more operation instructions to the wearable tremor control system 100. The housing 102 additionally includes a connection port 191 for transferring data, or transferring energy (e.g., to allow charging). The connection port 191 may comprise a USB port, USB Type-3, Thunderbolt, Thunderbolt 3, etc. Connectivity to the application 189 may be accomplished using one of several wireless technologies such as Bluetooth or wifi.

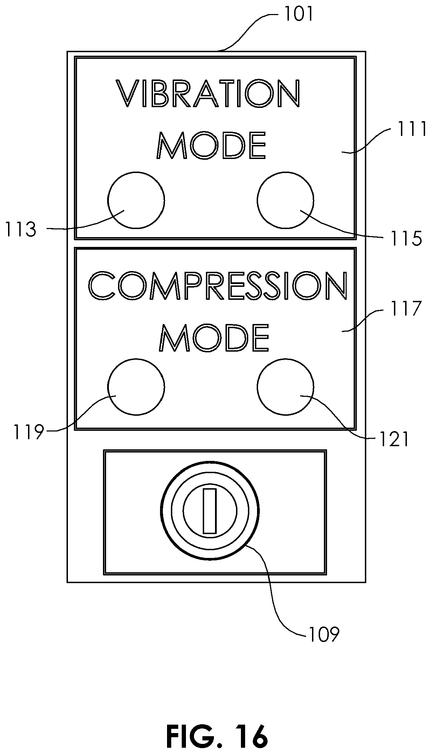

FIG. 16 illustrates the user interface 101 which includes a power switch 109 configured for turning the wearable tremor control system 100 on or off. The user interface 101 may comprise a touch screen, and may utilize capacitive or resistive touch sensitivity. Alternatively, mechanical or membrane buttons/switches may be utilized. A first control 111 having a first button 113 and a second button 115 is configured for manually adjusting the vibration mode. In other words, the vibration elements 136, 138 may be manually set (for example, to low vibration, medium vibration, or high vibration) using the first and/or second buttons 113, 115. Alternatively, the controller 192 and/or the App 189 may be configured (via software or firmware) to receive one or more signals from the sensing elements 132, 134, and to automatically adjust the vibration mode, either turning it on or off, or adjusting it between low, medium, and high vibration. The vibration mode in some embodiment may be automatically adjustable, via servo control or other methods, such that the vibration elements 136, 138 are caused to activate in a manner which is proportional to or matches in some way the reduction or increase in amplitude, intensity and/or prevalence of tremor. For example, the vibration elements 136, 138 may be configured to operate at a derived function of the dominant tremor frequency that is measured or calculated by the sensing elements 132, 134.

A second control 117 having a first button 119 and a second button 121 is configured for manually adjusting the compression mode. The inflation of the interior space 147 of the inner cuff 146 may be manually set (for example, to low inflation, medium inflation, or high inflation) using the first and/or second buttons 119, 121. Alternatively, the controller 192 and/or the App 189 may be configured (via software or firmware) to receive one or more signals from the sensing elements 132, 134, and to automatically adjust the compression mode, either turning it on or off, or adjusting it between low, medium, and high compression/spring normal force.

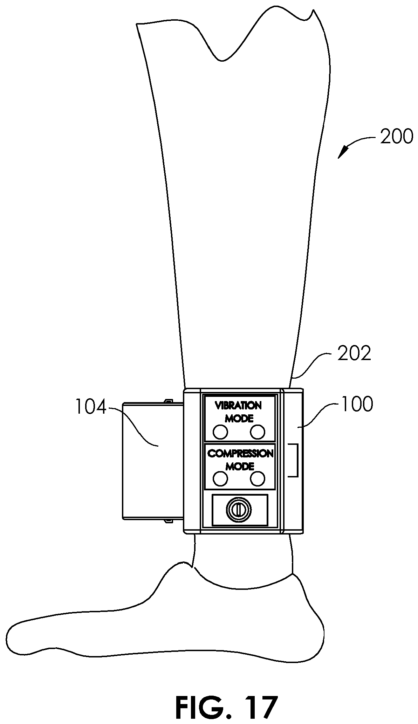

FIG. 17 illustrates a user 200 having restless leg syndrome (RLS) wearing the wearable tremor control system 100 in place around the ankle 202. The wearable tremor control system 100 may be worn by the user 200 while sleeping or resting in order to control involuntary movements of the leg(s) that affect RLS patients. All of the function of the wearable tremor control system 100 described in relation to the use on the wrist 38 of a user 42 may also be incorporated for a user 200 with RLS wearing the wearable tremor control system 100 on the ankle 202 or other portion of one or each leg.

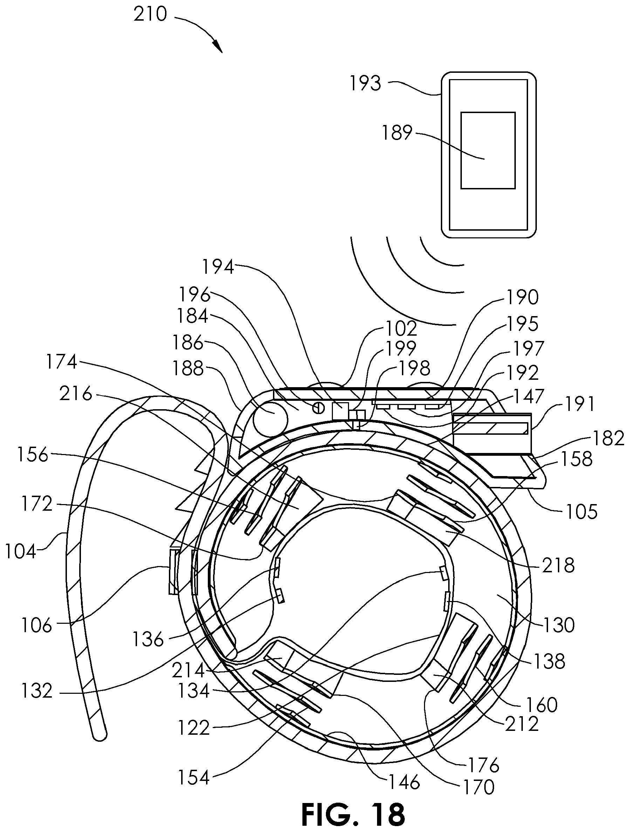

FIG. 18 illustrates a wearable tremor control system 210 which is similar to the wearable tremor control system 100 of FIG. 15, but which comprises additional weights 212, 214, 216, 218 coupled to the ends 170, 172, 174, 176 of each of the compression springs 154, 156, 158, 160. Each weight 212, 214, 216, 218 is capable of augmenting the forces placed on the wrist (limb, etc.) during compression by the inner cuff 146 inflation and normal force application be the compression springs 154, 156, 158, 160. As with the compression springs 154, 156, 158, 160, a force is applied at the "footprint" of each weight 212, 214, 216, 218, and thus in a focused area. Additionally, the compound weight (load) of the four weights 212, 214, 216, 218 together while carried by the band wearable tremor control system 210 serve as a constant static load that can further minimize the likelihood of tremors. The inertia of each weight 212, 214, 216, 218, alone and in combination with each other, serves to oppose motion of the limb initiated by involuntary muscular contractions. The applied forces will also be sensed in the brain of the user, which will alter limb shaking accordingly as part of a physiological feedback loop. The brain is thus "tricked" into playing a more involved interventional role. The weights 212, 214, 216, 218 may be made from high density materials, like lead, to allow a lower profile, and better fit within the closed band 104. Each weight may have a mass of between about 0.5 g to about 2 kg, or between about 10 g and about 500 g, or about 50 g to about 500 g, or about 10 g to about 250 g, or about 50 g and about 250 g. In some embodiments, wave springs may be used in place of the compression springs 154 ,156, 158, 160 to allow a smaller total profile of the wearable tremor control system 100, 210 on the wrist or ankle of the wearer. In other embodiments, the compression springs 154, 156, 158, 160 may be mounted on a rigid or semi-rigid backing having a footprint or area configured to amplify the compression force by a desired amount.

In one embodiment of the present disclosure, a system for treatment of involuntary muscle contraction comprises a wearable interface configured to at least partially encircle a first portion of a limb of a subject, a sensory module carried by the wearable interface and configured to output a signal related to muscular contraction within the limb, an energy application module carried by the wearable interface and configured to apply one or more forms of mechanical energy to the limb, wherein the energy application module is capable of changing the character of the one or more forms of mechanical energy in response to changes in the signal output from the sensory module. In some embodiments, the system for treatment of involuntary muscle contraction further comprises a controller configured to receive the signal output from the sensory module and to control changes in the character of at least one of the one or more forms of mechanical energy applied by the energy application module. In some embodiments, the controller is a microcontroller. The microcontroller may in some embodiments be an LFQP-100 microcontroller, and may include ARM (Advanced RISC Machine) architecture. In some embodiments, the controller is carried on the wearable interface, and may be electrically coupled to the sensory module (a sensor or an array of two or more sensors) and/or electrically coupled to the energy application module (an energy delivery element or an array of two or more energy delivery elements). In some embodiments, the wearable interface is in the form of a band, or watch, or bracelet. In some embodiments, the wearable interface is configured to completely encircle a limb (arm, leg) of the subject, at a portion such as a wrist or an ankle. In some embodiments, the wearable interface includes a closure device, such as a snap, a lock, a hook, a Velcro closure, a button closure, a snap closure, an adhesive closure, or a magnetic closure. In some embodiments, the sensory module comprises one or more piezoelectric elements, or one or more inflatable cuffs, or one or more non-inflatable cuffs, or one or more electrodes, or one or more displacement sensors, or one or more accelerometers, or one or more gyroscopes, or one or more electromyography (EMG) sensors. The one or more displacement sensors may comprise one or more piezo crystals. In some embodiments, the piezo crystal is configured to vibrate at at least a first frequency and a second frequency. In some embodiments, the first frequency is lower than the second frequency. In some embodiments, vibration at the first frequency is configured to at least partially dampen shaking of the limb of the subject, and vibration at the second frequency is configured to stimulate nerves in the limb of the subject. In some embodiments, the second frequency is a harmonic of the first frequency. In some embodiments, the piezo crystal is configured to vibrate at a frequency of between about 40 Hz and about 500 Hz, or between about 50 Hz and about 450 Hz, or between about 60 Hz and about 400 Hz, or between about 100 Hz and about 350 Hz. In some embodiments, the energy application module comprises one or more inflatable cuffs, or two or more inflatable cuffs. In some embodiments, each of the one or more or two or more inflatable cuffs is arrayed along a longitudinal axis of the limb of the subject when the wearable interface is in place on the first portion of the limb of the subject. In some embodiments, each of the one or more inflatable cuffs or two or more inflatable cuffs is arrayed along an external circumference of the limb of the subject when the wearable interface is in place on the first portion of the limb of the subject. In some embodiments, the energy application module comprises one or more weights. The one or more weights may be configured to apply a force against the limb of the subject. The one or more weights may be adjustable such that a force applied against the limb of the subject is variable. In some embodiments, one or more biasing members are coupled to one or more of the one or more weights. In some embodiments, the one or more biasing members comprise one or more helical elements, or one or more compression springs. In some embodiments, the one or more compression springs are configured to be adjustable such that a variable force is applied to the limb by the one or more weights. In some embodiments, the one or more compression springs are configured to be adjustable by inflation or deflation of at least a portion of the energy application module. In some embodiments, at least a portion of the energy application module comprises an inflatable cuff coupled to at least one of the one or more compression springs. In some embodiments, at least one of the one or more compression springs comprises an inflatable helical body. In some embodiments, each of the one or more weights has a mass of between about 0.5 gram and about 2,000 grams, or between about 10 grams and about 500 grams, or between about 10 grams and about 250 grams, or between about 50 grams and about 500 grams, or between about 50 grams and about 250 grams. In some embodiments, the energy application module comprises one or more ultrasound transducers. In some embodiments, the one or more ultrasound transducers are coupled to an inflatable cuff. In some embodiments, each of the one or more ultrasound transducers of the energy application module is configured to vibrate at a frequency of between about 1 Hz and about 30 Hz, or between about 2 Hz and about 15 Hz, or between about 3 Hz and about 10 Hz. In some embodiments, each of the one or more ultrasound transducers is configured to vibrate at a frequency of between about 15 kHz and about 1 MHz, or between about 20 kHz and about 700 kHz, or between about 25 kHZ and about 500 kHz, or between about 30 kHz and about 500 kHz, or between about 20 kHz and about 500 kHz, or between about 20 kHz and about 200 kHz, or between about 30 kHz and about 200 kHz, or between about 100 kHz and about 300 kHz.

In some embodiments, the character of the one or more forms of mechanical energy includes an amplitude of applied energy. In some embodiments, the character of the one or more forms of mechanical energy includes the orientation of geometry of the one or more forms of mechanical energy. In some embodiments, the character of the one or more forms of mechanical energy includes the ratio of the amount of energy applied by each of two or more of the one or more forms of mechanical energy. In some embodiments, the character of the one or more forms of mechanical energy includes the duration of application of the one or more forms of mechanical energy. In some embodiments, the character of the one or more forms of mechanical energy includes the duration of pauses between two or more applications of the one or more forms of mechanical energy. In some embodiments, the character of the one or more forms of mechanical energy includes the number of applications of the one or more forms of mechanical energy. In some embodiments, the energy application module is configured to increase the amplitude of energy applied to the limb in response to an increase in the amplitude of the signal output from the sensory module. In some embodiments, the energy application module is configured to decrease the amplitude of the energy applied to the limb in response to a decrease in the amplitude of the signal output from the sensory module. In some embodiments, the energy application module is configured to increase a frequency characteristic in energy applied to the limb in response to an increase in the amplitude of the signal output from the sensory module. In some embodiments, the energy application module is configured to decrease a frequency characteristic in energy applied to the limb in response to an increase in the amplitude of the signal output from the sensory module. A frequency characteristic may comprise a mean frequency of a wave of a particular type of energy.

In some embodiments, the sensory module is configured to output a signal related to Essential Tremor in the subject. In some embodiments, the sensory module is configured to output a signal related to Restless Leg Syndrome in the subject. In some embodiments, the sensory module is configured to output a signal related to Parkinson's Syndrome in the subject. In some embodiments, the sensory module is configured to output a signal related to Cerebellar Tremor in the subject. In some embodiments, the sensory module is configured to output a signal related to Dystonic Tremor in the subject. In some embodiments, the sensory module is configured to output a signal related to Action Tremor in the subject. In some embodiments, the sensory module is configured to output a signal related to Resting Tremor in the subject. In some embodiments, the sensory module is configured to output a signal related to one or more Psychological Disorders in the subject. In some embodiments, the sensory module is configured to be manually adjusted by a user. In some embodiments, the energy application module is configured to be manually adjusted by a user. In some embodiments, the controller is configured to be manually adjusted by a user. In some embodiments, the system for treatment of involuntary muscle contraction further comprises a battery carried on the wearable interface, and configured to power at least one of the sensory module or the energy application module. In some embodiments, the system for treatment of involuntary muscle contraction further comprises a communication module carried on the wearable interface and configured for wireless communication. In some embodiments, the communication module is configured to communicated with a mobile phone. In some embodiments, the system for treatment of involuntary muscle contraction further comprises a smart phone configured to run communication software capable of controlling communication with the communication module. In some embodiments, the communication software is firmware carried on the smart phone. In some embodiments, the communication software is a downloadable application. In some embodiments, at least one of the smart phone or the communications software provides a user interface for controlling operation of at least one element of the system for treatment of involuntary muscle contraction. In some embodiments, the communication module is configured to output data to the smart phone via the communication software. In some embodiments, communication between the communication module and the smart phone includes at least one security element. In some embodiments, the at least one security element includes encryption. In some embodiments, the at least one security element is password controlled.

In another embodiment of the present disclosure, a system for treatment of involuntary muscle contraction comprises a wearable interface configured to at least partially encircle a first portion of a limb of a subject, a sensory module carried by the wearable interface and configured to output a signal related to muscular contraction within the limb, and an energy application module comprising at least one compression element and at least one vibration element. In some embodiments, the system for treatment of involuntary muscle contraction further comprises a controller configured to receive the signal output from the sensory module and to control changes in the character of at least one or more forms of mechanical energy applied by the energy application module. In some embodiments, the controller is a microcontroller. The microcontroller may in some embodiments be an LFQP-100 microcontroller, and may include ARM (Advanced RISC Machine) architecture. In some embodiments, the controller is carried on the wearable interface, and may be electrically coupled to the sensory module (a sensor or an array of two or more sensors) and/or electrically coupled to the energy application module (an energy delivery element or an array of two or more energy delivery elements). In some embodiments, the at least one vibration element comprises at least one ultrasound transducer. In some embodiments, the wearable interface is in the form of a band, or watch, or bracelet. In some embodiments, the wearable interface is configured to completely encircle a limb (arm, leg) of the subject, at a portion such as a wrist or an ankle. In some embodiments, the wearable interface includes a closure device, such as a snap, a lock, a hook, a Velcro closure, a button closure, a snap closure, an adhesive closure, or a magnetic closure. In some embodiments, the sensory module comprises one or more piezoelectric elements, or one or more inflatable cuffs, or one or more non-inflatable cuffs, or one or more electrodes, or one or more displacement sensors, or one or more accelerometers, or one or more gyroscopes, or one or more electromyography (EMG) sensors. The one or more displacement sensors may comprise one or more piezo crystals. In some embodiments, the piezo crystal is configured to vibrate at at least a first frequency and a second frequency. In some embodiments, the energy application module comprises one or more inflatable cuffs, or two or more inflatable cuffs. In some embodiments, each of the one or more or two or more inflatable cuffs is arrayed along a longitudinal axis of the limb of the subject when the wearable interface is in place on the first portion of the limb of the subject. In some embodiments, each of the one or more inflatable cuffs or two or more inflatable cuffs is arrayed along an external circumference of the limb of the subject when the wearable interface is in place on the first portion of the limb of the subject. In some embodiments, the energy application module comprises one or more weights. The one or more weights may be configured to apply a force against the limb of the subject. The one or more weights may be adjustable such that a force applied against the limb of the subject is variable. In some embodiments, one or more biasing members are coupled to one or more of the one or more weights. In some embodiments, the one or more biasing members comprise one or more helical elements, or one or more compression springs. In some embodiments, the one or more compression springs are configured to be adjustable such that a variable force is applied to the limb by the one or more weights. In some embodiments, the one or more compression springs are configured to be adjustable by inflation or deflation of at least a portion of the energy application module. In some embodiments, at least a portion of the energy application module comprises an inflatable cuff coupled to at least one of the one or more compression springs. In some embodiments, at least one of the one or more compression springs comprises an inflatable helical body. In some embodiments, each of the one or more weights has a mass of between about 0.5 gram and about 2,000 grams, or between about 10 grams and about 500 grams, or between about 10 grams and about 250 grams, or between about 50 grams and about 500 grams, or between about 50 grams and about 250 grams. In some embodiments, the energy application module comprises one or more ultrasound transducers. In some embodiments, the one or more ultrasound transducers are coupled to an inflatable cuff. In some embodiments, each of the one or more ultrasound transducers of the energy application module is configured to vibrate at a frequency of between about 1 Hz and about 30 Hz, or between about 2 Hz and about 15 Hz, or between about 3 Hz and about 10 Hz. In some embodiments, each of the one or more ultrasound transducers of the energy application module is configured to vibrate at a frequency of between about 1 Hz and about 30 Hz, or between about 2 Hz and about 15 Hz, or between about 3 Hz and about 10 Hz. In some embodiments, each of the one or more ultrasound transducers is configured to vibrate at a frequency of between about 15 kHz and about 1 MHz, or between about 20 kHz and about 700 kHz, or between about 25 kHZ and about 500 kHz, or between about 30 kHz and about 500 kHz, or between about 20 kHz and about 500 kHz, or between about 20 kHz and about 200 kHz, or between about 30 kHz and about 200 kHz, or between about 100 kHz and about 300 kHz.

In some embodiments, the character of the one or more forms of mechanical energy includes an amplitude of applied energy. In some embodiments, the character of the one or more forms of mechanical energy includes the orientation of geometry of the one or more forms of mechanical energy. In some embodiments, the character of the one or more forms of mechanical energy includes the ratio of the amount of energy applied by each of two or more of the one or more forms of mechanical energy. In some embodiments, the character of the one or more forms of mechanical energy includes the duration of application of the one or more forms of mechanical energy. In some embodiments, the character of the one or more forms of mechanical energy includes the duration of pauses between two or more applications of the one or more forms of mechanical energy. In some embodiments, the character of the one or more forms of mechanical energy includes the number of applications of the one or more forms of mechanical energy. In some embodiments, the energy application module is configured to increase the amplitude of energy applied to the limb in response to an increase in the amplitude of the signal output from the sensory module. In some embodiments, the energy application module is configured to decrease the amplitude of the energy applied to the limb in response to a decrease in the amplitude of the signal output from the sensory module. In some embodiments, the energy application module is configured to increase a frequency characteristic in energy applied to the limb in response to an increase in the amplitude of the signal output from the sensory module. In some embodiments, the energy application module is configured to decrease a frequency characteristic in energy applied to the limb in response to an increase in the amplitude of the signal output from the sensory module. A frequency characteristic may comprise a mean frequency of a wave of a particular type of energy.

In some embodiments, the sensory module is configured to output a signal related to Essential Tremor in the subject. In some embodiments, the sensory module is configured to output a signal related to Restless Leg Syndrome in the subject. In some embodiments, the sensory module is configured to output a signal related to Parkinson's Syndrome in the subject. In some embodiments, the sensory module is configured to output a signal related to Cerebellar Tremor in the subject. In some embodiments, the sensory module is configured to output a signal related to Dystonic Tremor in the subject. In some embodiments, the sensory module is configured to output a signal related to Action Tremor in the subject. In some embodiments, the sensory module is configured to output a signal related to Resting Tremor in the subject. In some embodiments, the sensory module is configured to output a signal related to one or more Psychological Disorders in the subject. In some embodiments, the sensory module is configured to be manually adjusted by a user. In some embodiments, the energy application module is configured to be manually adjusted by a user. In some embodiments, the controller is configured to be manually adjusted by a user. In some embodiments, the system for treatment of involuntary muscle contraction further comprises a battery carried on the wearable interface, and configured to power at least one of the sensory module or the energy application module. In some embodiments, the system for treatment of involuntary muscle contraction further comprises a communication module carried on the wearable interface and configured for wireless communication. In some embodiments, the communication module is configured to communicated with a mobile phone. In some embodiments, the system for treatment of involuntary muscle contraction further comprises a smart phone configured to run communication software capable of controlling communication with the communication module. In some embodiments, the communication software is firmware carried on the smart phone. In some embodiments, the communication software is a downloadable application. In some embodiments, at least one of the smart phone or the communications software provides a user interface for controlling operation of at least one element of the system for treatment of involuntary muscle contraction. In some embodiments, the communication module is configured to output data to the smart phone via the communication software. In some embodiments, communication between the communication module and the smart phone includes at least one security element. In some embodiments, the at least one security element includes encryption. In some embodiments, the at least one security element is password controlled.

In another embodiment of the present disclosure, a system for treatment of involuntary muscle contraction comprises a wearable interface configured to at least partially encircle a first portion of a limb of a subject, and an energy application module comprising at least one compression element and at least one ultrasound transducer. In some embodiments, the compression element comprises at least one weight. In some embodiments, the compression element comprises at least one inflatable cuff. In some embodiments, the system for treatment of involuntary muscle contraction further comprises a controller configured to control changes in the character of at least one of one or more forms of mechanical energy applied by the energy application module. In some embodiments, the controller is a microcontroller. The microcontroller may in some embodiments be an LFQP-100 microcontroller, and may include ARM (Advanced RISC Machine) architecture. In some embodiments, the controller is carried on the wearable interface, and may be electrically coupled to the sensory module (a sensor or an array of two or more sensors) and/or electrically coupled to the energy application module (an energy delivery element or an array of two or more energy delivery elements). In some embodiments, the at least one vibration element comprises at least one ultrasound transducer. In some embodiments, the wearable interface is in the form of a band, or watch, or bracelet. In some embodiments, the wearable interface is configured to completely encircle a limb (arm, leg) of the subject, at a portion such as a wrist or an ankle. In some embodiments, the wearable interface includes a closure device, such as a snap, a lock, a hook, a Velcro closure, a button closure, a snap closure, an adhesive closure, or a magnetic closure. In some embodiments, the system for treatment of involuntary muscle contraction further comprises a communication module carried on the wearable interface and configured for wireless communication. In some embodiments, the communication module is configured to communicated with a mobile phone. In some embodiments, the system for treatment of involuntary muscle contraction further comprises a smart phone configured to run communication software capable of controlling communication with the communication module. In some embodiments, the communication software is firmware carried on the smart phone. In some embodiments, the communication software is a downloadable application. In some embodiments, at least one of the smart phone or the communications software provides a user interface for controlling operation of at least one element of the system for treatment of involuntary muscle contraction. In some embodiments, the communication module is configured to output data to the smart phone via the communication software. In some embodiments, communication between the communication module and the smart phone includes at least one security element. In some embodiments, the at least one security element includes encryption. In some embodiments, the at least one security element is password controlled.

In another embodiment of the present disclosure, a system for treatment of involuntary muscle contraction comprises a wearable interface configured to at least partially encircle a first portion of a limb of a subject, and an energy application module comprising at least one compression element comprising one or more adjustable weights. In some embodiments, the system for treatment of involuntary muscle contraction further comprises at least one vibration element. In some embodiments, the system for treatment of involuntary muscle contraction further comprises a controller configured to control changes in the character of at least one of one or more forms of mechanical energy applied by the energy application module. In some embodiments, the controller is a microcontroller. The microcontroller may in some embodiments be an LFQP-100 microcontroller, and may include ARM (Advanced RISC Machine) architecture. In some embodiments, the controller is carried on the wearable interface, and may be electrically coupled to the energy application module. In some embodiments, the at least one vibration element comprises at least one ultrasound transducer. In some embodiments, the wearable interface is in the form of a band, or watch, or bracelet. In some embodiments, the wearable interface is configured to completely encircle a limb (arm, leg) of the subject, at a portion such as a wrist or an ankle. In some embodiments, the wearable interface includes a closure device, such as a snap, a lock, a hook, a Velcro closure, a button closure, a snap closure, an adhesive closure, or a magnetic closure. In some embodiments, the system for treatment of involuntary muscle contraction further comprises a communication module carried on the wearable interface and configured for wireless communication. In some embodiments, the communication module is configured to communicated with a mobile phone. In some embodiments, the system for treatment of involuntary muscle contraction further comprises a smart phone configured to run communication software capable of controlling communication with the communication module. In some embodiments, the communication software is firmware carried on the smart phone. In some embodiments, the communication software is a downloadable application. In some embodiments, at least one of the smart phone or the communications software provides a user interface for controlling operation of at least one element of the system for treatment of involuntary muscle contraction. In some embodiments, the communication module is configured to output data to the smart phone via the communication software. In some embodiments, communication between the communication module and the smart phone includes at least one security element. In some embodiments, the at least one security element includes encryption. In some embodiments, the at least one security element is password controlled.

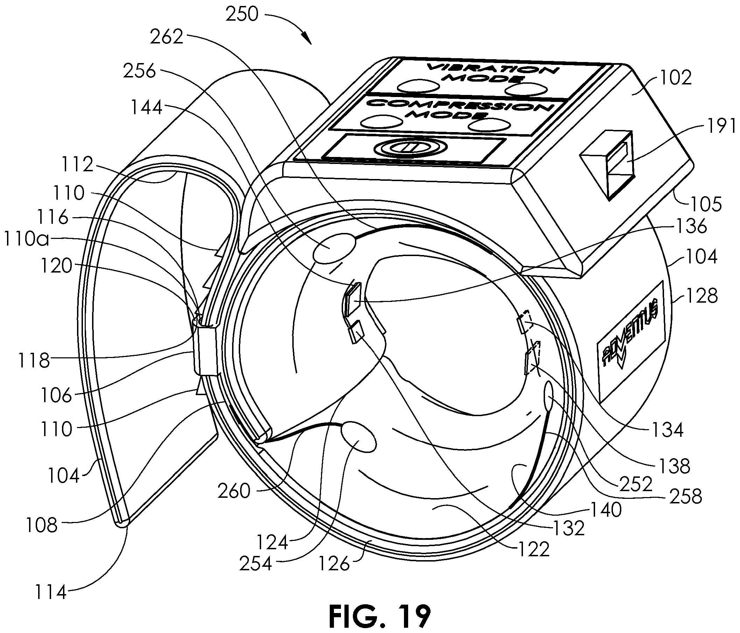

FIG. 19 illustrates a wearable tremor control system 250 that is similar to the wearable tremor control system 100 of FIG. 8, but additionally comprises stimulation electrodes 252, 254, 256 carried on the outer surface 140 of the outer cuff 122. The user interface 101 and/or app 189 may be configured to adjust or program the controller 192 such that signals received from the one or more signals from the sensing elements 132, 134 cause current to run through wires or traces 258, 260, 262 electrically coupled to the electrodes 252, 254, 256, thus applying one or more potentials (voltages) across two or more of the electrodes. In some embodiments, a current may be applied using voltage control. In some embodiments, a current may also be applied using current control. The applied current is capable of activating nerves, for example, to provide an additional input to the brain. The user interface 101 (FIG. 16) may include a third mode that is an electrical stimulation mode, also capable of being adjusted manually (e.g., with two buttons), or with feedback from the sensing elements 132, 134. Any combination of two or three (or more) modes may be possible, or in some embodiments, only a single mode. The electrodes 252, 254, 256 may be configured such that the one or more applied potentials are directed to the median nerve to thereby stimulate it in order to alter or induce the brain's control or modification of tremors. In alternative embodiments, the electrodes 252, 254, 256 may be configured to sense physiological signals related to tremors. The controller 192 may be configured or programmable to be configured, via hardware, firmware, or software, such that any one or more of the inflation of the inner cuff 146, activation of the electrodes 252, 254, 256, or activation of the vibration elements 136, 138 is applied with a particular range of set parameters or set parameter ranges, thus serving as a programmable pulse generator. For example, in certain embodiments, the voltage, current, frequency, or pulse width of the activation of the electrodes 252, 254, 256 may be controlled within the following ranges. Current: 0.1 mA to 200 mA, or 0.1 mA to 50 mA; frequency/rate of application: 0.1 mA to 200 mA, or 1 Hz to 5,000 Hz, or 1 Hz to 1,000 Hz, or 1 Hz to 200 Hz; pulse width: 0.01 microsecond (µs) to 1000 microseconds (µs), or 1 microsecond (µs) to 1000 microseconds (µs), or 0.01 microsecond (µs) to 5 microseconds (µs). The controller 192 may fire the electrodes in a continuous mode, or in random mode comprising one or more bursts. The one-time of the bursts and the off-time of the bursts may each be independently controlled. A particular program or algorithm may be used to vary the on-times and off-times. In alternative embodiments, the controller 192 may be configured or programmable to be configured, via hardware, firmware, or software, such that any one or more of the inflation of the inner cuff 146, activation of the electrodes 252, 254, 256, or activation of the vibration elements 136, 138 is applied in an at least partially random or pseudo-random manner. The human body is adaptable, and, like many physiological systems, tends to adjust to therapeutic treatments, sometimes in a manner that, to the body, appears helpful, when in fact it is antagonistic to the purposes or effects of treatment. Nervous systems are able to continually change by processes such as synaptic adaptation. These changes may actually decrease the effectiveness of an initially effective treatment over time. Thus, by adding random changes to the way the therapeutic elements (inner cuff 146/ compression springs 154, 156, 158, 160/weights 212, 214, 216, 218; vibration elements 136, 138; electrodes 252, 254, 256) are applied can serve as a way of getting ahead of or "tricking" the body's adaptation schemes that may otherwise actually prove antagonistic to efforts to minimize the effects of tremors. Parameters that may be adjusted, randomly, or non-randomly, by the controller 192 include: time of application of energy (mechanical, electrical, etc.), length of interval of time between application of energy, number of repetitions of application of energy, particular operational frequency of a non-static mode of energy (e.g., applying ultrasound at varying pulse rates), amplitude of the applied energy, timing of particular combinations of more than one element of a particular type of energy, or of two or more different types of energy. Any of these parameters can be increased or decreased. The controller 192 may be configured to allow the user/patient to control some or all of these parameter adjustments, for example, via the user interface 101 and/or app 189. In addition, in some embodiments, there may be security levels to control how much the user can or cannot control, for example, a first level for a user and a second level for a prescribing physician. In some embodiments, the existence of controls available to the physician that are not available to the user may assure a certain amount of randomness in the treatment. This may even be necessary in some cases, for example, for particular patients that do not want to be surprised with a compression, electrode firing, or vibration event. The security levels may include encryption and/or password control. The "smart" nature of the wearable tremor control system 250, or any of the other systems described in the embodiments herein, allows it to be managed by primary care physicians, ad thus, not requiring a specialist.

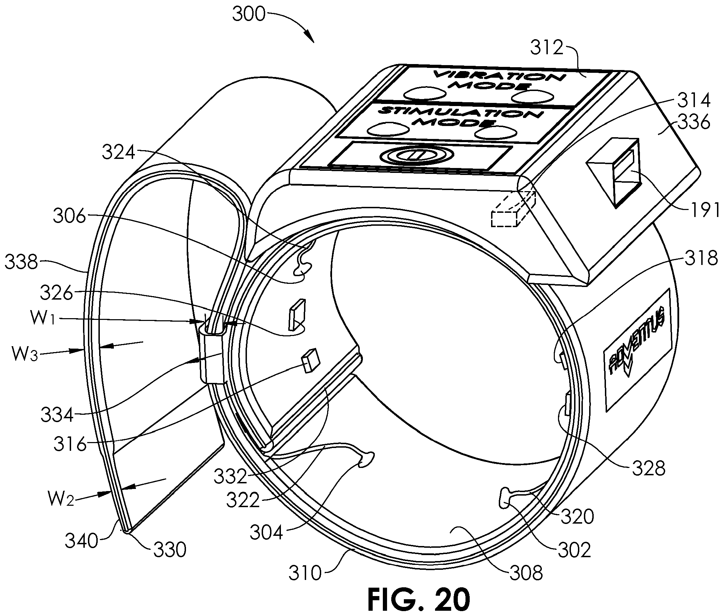

FIG. 20 illustrates a wearable tremor control system 300 having multi-mode energy delivery therapy including both vibration and electrical stimulation. The wearable tremor control system 300 is similar to the wearable tremor control system 100 of FIG. 8, but does not include compression, and does comprise stimulation electrodes 302, 304, 306 carried on the limb-facing surface 308 of the band 310. The band 310 has a first end 330 and a second end 332 and is removably attached to a removable/replaceable housing 336. A loop 334 is secured to the band 310, and has an opening width W₁. An insertion section 340 of the band 310 has a thickness W₂ that is less than opening width W₁. The normal wall 338 of the band 310 has a thickness W₃ that is slightly greater than the opening width W₁, thus creating a friction fit, which renders wedges 110 or hook/loop 20a/20b unnecessary. In use, a user places the band 310 around the target limb, inserts the insertion section 340 into the loop 334, and pulls the band 310 from the first end 330 until adjusted to an acceptable amount on the limb of the user/wearer. The friction between the normal wall 338 and the loop 334 maintains the band 310 secure. The loop 334 may comprise an elastomeric material, such as a rubber or a elastomer, or a thermoplastic elastomer, to allow the loop to stretch when the normal wall 338 portion of the band 310 is placed through it. The user interface 312 and/or app 189 (FIGS. 15 or 18) may be configured to adjust or program the controller 314 such that signals received from the one or more signals from the sensing elements 316, 318 cause current to run through wires or traces 320, 322, 324 electrically coupled to the electrodes 302, 304, 306, thus applying one or more potentials (voltages) across two or more of the electrodes. A current may be applied using voltage control. A current may also be applied using current control. The applied current is capable of activating nerves, for example, to provide an additional input to the brain. The user interface 312 includes a vibration mode (for activating vibration elements 326, 328) and a stimulation mode (via electrode(s) 302, 304, 306), which are each capable of being adjusted manually, or with feedback from the sensing elements 316, 318. Any combination of the two modes may be possible, such that a mixed signal may be created. The mixed signal may include a cycle having a first period of only one of vibration or stimulation and a second period of the other of vibration or stimulation. The mixed signal may also include at least one period of simultaneous vibration and stimulation. The mixed signal may include a first period with both vibration and stimulation and a second period with both vibration and stimulation, wherein the ratio between the amount of vibration and stimulation (energy or amplitude, etc.) is different between the first period and second period. The electrodes 302, 304, 306 may be configured such that the one or more applied potentials are directed to the median nerve to thereby stimulate it in order to alter or induce the brain's control or modification of tremors. In alternative embodiments, the electrodes 302, 304, 306 may be configured to sense physiological signals related to tremors. The controller 314 may be configured or programmable to be configured, via hardware, firmware, or software, such that any one or more of the activation of the electrodes 302, 304, 306 or activation of the vibration elements 326, 328 is applied with a particular range of set parameters or set parameter ranges, thus serving as a programmable pulse generator. For example, in certain embodiments, the voltage, current, frequency, or pulse width of the activation of the electrodes 302, 304, 306 may be controlled within the following ranges. Current: 0.1 mA to 200 mA, or 0.1 mA to 50 mA; frequency/rate of application: 0.01 Hz to 50 kHz, or 1 Hz to 5,000 Hz, or 1 Hz to 1,000 Hz, or 1 Hz to 200 Hz; pulse width: 1 microsecond (µs) to 1000 milliseconds (µs), or 1 microsecond (µs) to 1000 microseconds (µs), or 0.01 millisecond (ms) to 5 milliseconds (ms). The controller 314 may fire the electrodes in a continuous mode, or in random mode comprising one or more bursts. The period of activation of the electrodes 302, 304, 306 may include one or more of the following patterns: a biphasic sine wave, a multiphasic wave, a monophasic sine wave, a biphasic pulsatile sine wave, a biphasic rectangular wave, a monophasic square wave, a monophasic pulsatile rectangular wave, a biphasic spiked wave, a monophasic spiked wave, and a monophasic pulsatile spiked wave. The one-time of the bursts and the off-time of the bursts may each be independently controlled. A particular program or algorithm may be used to vary the on-times and off-times. In alternative embodiments, the controller 314 may be configured or programmable to be configured, via hardware, firmware, or software, such that any one or more of the activation of the electrodes 302, 304, 306 or activation of the vibration elements 326, 328 is applied in an at least partially random or pseudo-random manner, as described in relation for the embodiment of FIG. 19. Any one of the electrodes 302, 304, 306 may serve as a patient return electrode, thus making unnecessary an additional skin-placed return electrode patch. Thus, the simple coupling of the band 310 on the limb of the wearer/user allows the wearer/user to immediately begin using the wearable tremor control system 300.

Multiple touch points are provided by the electrodes 302, 304, 306 and vibration elements 326, 328, which are located at different clock locations around the limb-facing surface 308 of the band 310, thus allowing for a high success rate, as an optimal anatomical location for effective therapy is more likely to be identified and treated. The electrodes 302, 304, 306 and the vibration elements 326, 328 can be controlled by the controller 314 to work in synchrony to deliver optimal results. The controller 314 may be configured to allow the user/patient to control some or all of these parameter adjustments, for example, via the user interface 312 and/or app 189. In addition, in some embodiments, there may be security levels to control how much the user can control: a first level for a user and a second level for a prescribing physician. In some embodiments, the existence of controls available to the physician that are not available to the user may assure a certain amount of randomness in the treatment. This may even be necessary in some cases, for example, for particular patients that do not want to be surprised with an electrode firing, or vibration event. The security levels may include encryption and/or password control. Many of the components described in the wearable tremor control system 300 have relatively low power requirements, thus being amenable to a chargeable battery system. The connection port 191 may also be used to attached a wireless antenna, if needed, whether or not there is internal wireless capability within the wearable tremor control system 300.

The wearable tremor control system 300 may include adaptive capabilities. For example, the controller 314 may be programmable, or pre-programmed, to provide a particular therapy plan, such as a morning application of energy, a mid-day application of energy, and an evening application of energy. However, by analyzing physiological activity measured by the sensing elements 316, 318, the controller 314 may be configured to change the therapy plan to optimize patient response. For example, the change may include a larger amplitude and/or longer duration of the application of vibrational energy and a smaller amplitude and/or shorter duration of the application of electrical stimulation energy. Or, in other cases, the change may include a larger amplitude and/or longer duration of the application of electrical stimulation energy and a smaller amplitude and/or shorter duration of the application of vibrational energy. An energy modulation algorithm may be applied, allowing the wearable tremor control system 300 to learn and better deliver custom neuromodulation management to each wearer, which may correspond to each patient's particular tremor symptoms. Thus, an individualized treatment plan may be constructed or adapted for each patient/user.

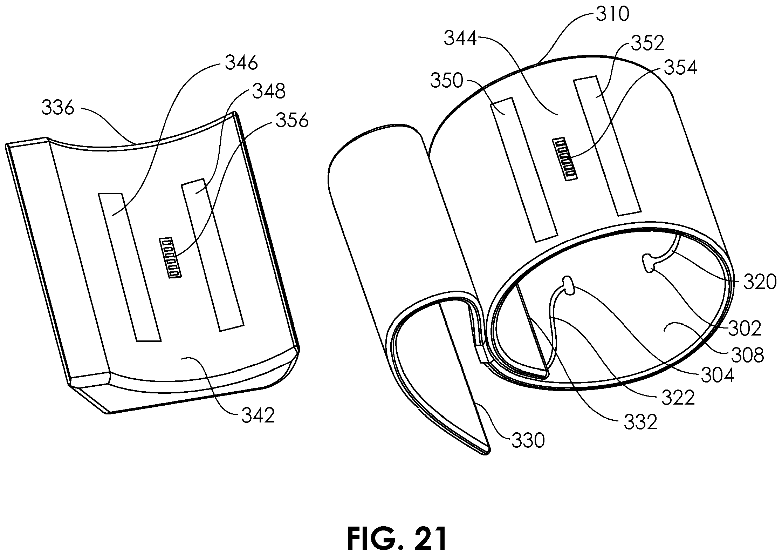

In FIG. 21, the housing 336 has been removed from the band 310. The housing 336 is removeable from and reattachable to the band 310 for multiple reasons. The housing 336 may include one or more rechargeable batteries which can be recharged by attachment of a power cable to the connection port 191 (FIG. 20), or to another port, connected to the batteries. The batteries may be rechargeable by wired or by wireless methods, including inductively-coupled charging. The one or more batteries may be similar to the battery 186 of FIGS. 12-15. In alternative embodiments, one or more of the batteries may be a primary cell, configured to be used and discarded (or recycled). The housing 336 is secured to the band 310 via two magnets 346, 348 which are configured to attract magnets 350, 352 carried on the band 310. In the embodiment of FIG. 21, magnet 348 has an externally-facing positive pole which is configured to magnetically engage with magnet 350, which has an externally-facing negative pole. Magnet 346 has an externally-facing negative pole which is configured to magnetically engage with magnet 352, which has an externally-facing positive pole. The magnets may comprise rare earth magnets, such as neodymium-iron-boron or samarium cobalt. The neodymium-iron-boron magnets may be chosen from a grade of N30 or higher, or N33 or higher, or N35 or higher, or N38 or higher, or N40 or higher, or N42 or higher, or N45 or higher, or N48 or higher, or N50 or higher. In some embodiments, the neodymium-iron-boron magnets may have a grade between N30 and N52, or between N33 and N50 or between N35 and N48.

Electrical connection may be achieved by conductive projections 354 carried on the band 310 and which are configured to conductively engage with conductive depressions 356 carried on the bottom surface 342 of the housing 336. The conductive depressions 356 are electrically connected to the various components of the housing 336, which may include the user interface 312, the controller 314, and the connection port 191, or any of the electrical components described in the prior embodiments. The conductive projections 354 are electrically connected to the traces 320, 322, 324 and stimulation electrodes 302, 304, 306, the vibration elements 326, 328, and the sensing elements 316, 318 (FIG. 20). Thus, when the housing 336 is attached to the band 310 via the attraction of the magnets 346, 348, 350, 352, the conductive projections 354 are electrically coupled to the conductive depressions 356. They may comprise a terminal connection featuring pins and holes. The user interface 312, the controller 314, the connection port 191, and other electrical components are thereby electrically interlinked with the traces 320, 322, 324 and stimulation electrodes 302, 304, 306, the vibration elements 326, 328, and the sensing elements 316, 318. A user may choose to remove the housing 336 from the band 310 for other reasons than recharging. For example, a first housing 336 may be replaced by a second housing 336, if the first housing 336 is damaged or ceases to function. The housing 336 may be removed to present to a medical facility, which may upload or download information or software revisions, or for maintenance or repair. The conductive projections 354 and the conductive depressions 356 are shown in FIG. 21 located between the magnets 350, 352 or magnets 346, 348, respectively, but in other embodiments, the conductive projections 354 are electrically coupled to the conductive depressions 356 may be located laterally from the magnets 350, 352 or magnets 346, 348. In some embodiments, the conductive projections 354 and conductive depressions 356 may each be replaced by a series of conductive terminals that each have both projections and depressions, or by a series of terminals that have a substantially planar array of conductive terminals (neither projections nor depressions).

In alternative embodiments the magnets 350, 352 may be replaced by ferrous metal strips, which will also be attracted to the magnets 346, 348. Or, the magnets 346, 348 can instead be replaced by ferrous metal strips, instead of the magnets 350, 352. In other alternative embodiments, the magnets 346, 348, 350, 352 may be substituted by other connections, such as snaps, hooks-and-loops (Velcro®), sliding engagements, or adhesive strips.

In one embodiment of the present disclosure, a system for treatment of involuntary muscle contraction comprises a wearable interface having an internal contact face, the wearable interface configured to at least partially encircle a first portion of a limb of a subject, one or more weights carried by the wearable interface, and one or more electrodes carried on the internal contact face and configured to contact the skin within the first portion of the limb of the subject. In some embodiments, the system for treatment of involuntary muscle contraction further comprises a controller configured to energize the one or more electrodes. In some embodiments, the one or more electrodes are configured to apply one or more impulses to stimulate the median nerve of an upper limb of the subject. In some embodiments, the controller is configured to apply the impulses by the electrodes in a random pattern. In some embodiments, the random pattern comprises randomly varying time periods between consecutive series of impulses. In some embodiments, the random pattern comprises randomly varying an operating frequency between one series of impulses and another series of impulses.

In another embodiment of the present disclosure, a system for treatment of involuntary muscle contraction comprises a wearable interface having an internal contact surface, the wearable interface configured to at least partially encircle a first portion of a limb of a subject, one or more electrodes carried on the internal contact surface and configured to contact the skin within the first portion of the limb of the subject, and a control unit configured to control the activation of the one or more electrodes. In some embodiments, the control unit is programmable. In some embodiments, the control unit comprises a microcontroller. The microcontroller may in some embodiments be an LFQP-100 microcontroller, and may include ARM (Advanced RISC Machine) architecture. In some embodiments, the control unit is configured to at least partially define a pulse of activation of the electrode. In some embodiments, the control unit is configured to be programmed to activate the one or more electrodes to apply a current of between about 0.1 mA and about 50 mA. In some embodiments, the control unit is configured to be programmed to pulse the one or more electrodes at a rate of between about 1 Hz and about 5,000 Hz, or between about 1 Hz and about 1,000 Hz, or between about 1 Hz and about 200 Hz. In some embodiments, the control unit is configured to be programmed to activate the one or more electrodes at a pulse having a pulse width of between about 1 µs to about 1,000 µs. In some embodiments, the control unit is configured to be able to control at least one of the on-time and the off-time of the pulse. In some embodiments, the control unit is configured to activate the one or more electrodes in a random or pseudo-random manner. In some embodiments, the control unit is configured to activate the one or more electrodes to stimulate the median nerve of an upper limb of the subject.

In another embodiment of the present disclosure, a system for treatment of involuntary muscle contraction comprises a wearable interface having an internal contact surface, the wearable interface configured to at least partially encircle a first portion of a limb of a subject, one or more electrodes carried on the internal contact surface and configured to contact the skin within the first portion of the limb of the subject, an energy application module comprising at least one vibration element, and a control unit configured to control the activation of the one or more electrodes. In some embodiments, the system for treatment of involuntary muscle contraction further comprises a sensory module carried by the wearable interface and configured to output a signal related to muscular contraction within the limb. In some embodiments, the control unit is programmable. In some embodiments, the control unit is further configured to control activation of the at least one vibration unit. In some embodiments, the control unit comprises a microcontroller. The microcontroller may in some embodiments be an LFQP-100 microcontroller, and may include ARM (Advanced RISC Machine) architecture. In some embodiments, the control units is configured to cause the activation of the one or more electrodes and the at least one vibration element to together create a mixed signal. In some embodiments, the control unit is configured to produce an activation cycle comprising a first period of activation of the at least one vibration element without activation of the one or more electrodes, and a second period activation of the one or more electrodes. The second period of activation, in some embodiments, includes activation of the at least one vibration element. The second period of activation of the one or more electrode, in some embodiments, comprises continuous activation. The second period of activation of the one or more electrodes, in some embodiments, comprises pulsed activation. The second period of activation of the one or more electrodes, in some embodiments, comprises a sinusoidal wave. The second period of activation of the one or more electrodes, in some embodiments, comprises a square wave. The second period of activation of the one or more electrodes, in some embodiments, comprises at least one of the patterns in the list consisting of: a biphasic sine wave, a multiphasic wave, a monophasic sine wave, a biphasic pulsatile sine wave, a biphasic rectangular wave, a monophasic square wave, a monophasic pulsatile rectangular wave, a biphasic spiked wave, a monophasic spiked wave, and a monophasic pulsatile spiked wave. In some embodiments, the control unit is configured to repeat the activation cycle one or more times. In some embodiments, the control unit is configured to at least partially define a pulse of activation of the electrode. In some embodiments, the control unit is configured to be programmed to activate the one or more electrodes to apply a current of between about 0.1 mA and about 200 mA. In some embodiments, the control unit is configured to be programmed to pulse the one or more electrodes at a rate of between about 0.01 Hz and about 5,000 Hz, or between about 0.01 Hz and about 1,000 Hz, or between about 0.01 Hz and about 5 Hz. In some embodiments, the control unit is configured to be programmed to activate the one or more electrodes at a pulse having a pulse width of between about 0.01 millisecond to about 5 milliseconds. In some embodiments, the control unit is configured to control at least one of the on-time and the off-time of a pulse. In some embodiments, the control unit is configured to activate the one or more electrodes in a random or pseudo-random manner. In some embodiments, the control unit is configured to activate the one or more electrodes to stimulate the median nerve of an upper limb of the subject.

In some embodiments, the sensory module comprises at least one piezo crystal. In some embodiments, the at least one piezo crystal is configured to vibrate at a frequency of between about 40 Hz and about 500 Hz, or between about 50 Hz and about 450 Hz, or between about 60 Hz and about 400 Hz, or between about 100 Hz and about 350 Hz. In some embodiments, the control unit is carried by the wearable interface. In some embodiments, the wearable interface is in the form of a band, or watch, or bracelet. In some embodiments, the wearable interface is configured to completely encircle a limb (arm, leg) of the subject, at a portion such as a wrist or an ankle. In some embodiments, the wearable interface includes a closure device, such as a snap, a lock, a hook, a Velcro closure, a button closure, a snap closure, an adhesive closure, or a magnetic closure. In some embodiments, the control unit is configured to control the activation of the at least one vibration element at a first frequency and at a second frequency. In some embodiments, the first frequency is lower than the second frequency. In some embodiments, vibration at the first frequency is configured to at least partially dampen shaking of the limb of the subject, and vibration at the second frequency is configured to stimulate nerves in the limb of the subject. In some embodiments, the second frequency is a harmonic of the first frequency. In some embodiments, the system for treatment of involuntary muscle contraction further comprises a communication module carried on the wearable interface and configured for wireless communication. In some embodiments, the communication module is configured to communicated with a mobile phone. In some embodiments, the system for treatment of involuntary muscle contraction further comprises a smart phone configured to run communication software capable of controlling communication with the communication module. In some embodiments, the communication software is firmware carried on the smart phone. In some embodiments, the communication software is a downloadable application. In some embodiments, at least one of the smart phone or the communications software provides a user interface for controlling operation of at least one element of the system for treatment of involuntary muscle contraction. In some embodiments, the communication module is configured to output data to the smart phone via the communication software. In some embodiments, communication between the communication module and the smart phone includes at least one security element. In some embodiments, the at least one security element includes encryption. In some embodiments, the at least one security element is password controlled.

In some embodiments, the sensory module is configured to output a signal related to Essential Tremor in the subject. In some embodiments, the sensory module is configured to output a signal related to Restless Leg Syndrome in the subject. In some embodiments, the sensory module is configured to output a signal related to Parkinson's Syndrome in the subject. In some embodiments, the sensory module is configured to output a signal related to Cerebellar Tremor in the subject. In some embodiments, the sensory module is configured to output a signal related to Dystonic Tremor in the subject. In some embodiments, the sensory module is configured to output a signal related to Action Tremor in the subject. In some embodiments, the sensory module is configured to output a signal related to Resting Tremor in the subject. In some embodiments, the sensory module is configured to output a signal related to one or more Psychological Disorders in the subject.

In some embodiments, the control unit is carried on a housing, the housing configured to be removably secured to the at least one of a band, a watch, or a bracelet. In some embodiments, the housing includes a first coupling member and the at least one of a band, a watch, or a bracelet includes a second coupling member, the first coupling member and second coupling member attachable to and detachable from each other. In some embodiments, at least one of the first coupling member or the second coupling member comprises a magnet. In some embodiments, one of the first coupling member or second coupling member comprises a magnet and the other of the first coupling member or second coupling member comprises a ferrous metal. In some embodiments, the first coupling member comprises a first magnet and the second coupling member comprises a second magnet. In some embodiments, a north pole of one of the first magnet or second magnet is configured to magnetically interface with a south pole of the other of the first coupling member or second coupling member. In some embodiments, the housing includes a third coupling member and the at least one of a band, a watch, or a bracelet includes a fourth coupling member, the third coupling member and fourth coupling member attachable to and detachable from each other. In some embodiments, at least one of the third coupling member or the fourth coupling member comprises a magnet. In some embodiments, one of the third coupling member or fourth coupling member comprises a magnet and the other of the third coupling member or fourth coupling member comprises a ferrous metal. In some embodiments, the third coupling member comprises a third magnet and the fourth coupling member comprises a fourth magnet. In some embodiments, a north pole of one of the third magnet or fourth magnet is configured to magnetically interface with a south pole of the other of the third coupling member or fourth coupling member. In some embodiments, the first coupling member and second coupling member comprise snaps. In some embodiments, the first coupling member and second coupling member comprise a hook and loop system. In some embodiments, one of the first coupling member and second coupling member comprises a channel and the other of the first coupling member and second coupling member comprises a projection configured to lock within the channel. In some embodiments, at least one of the housing or the at least one of a band, a watch, or a bracelet includes a proximity sensor, configured to output a signal when the housing is secured to the at least one of a band, a watch, or a bracelet. In some embodiments, the proximity sensor comprises a Hall-effect device.

In some embodiments, the at least one vibration element comprises one or more ultrasound transducers configured to vibrate at a frequency of between about 15 kHz and about 1 MHz, or between about 20 kHz and about 700 kHz, or between about 25 kHZ and about 500 kHz, or between about 30 kHz and about 500 kHz, or between about 20 kHz and about 500 kHz, or between about 20 kHz and about 200 kHz, or between about 30 kHz and about 200 kHz, or between about 100 kHz and about 300 kHz.

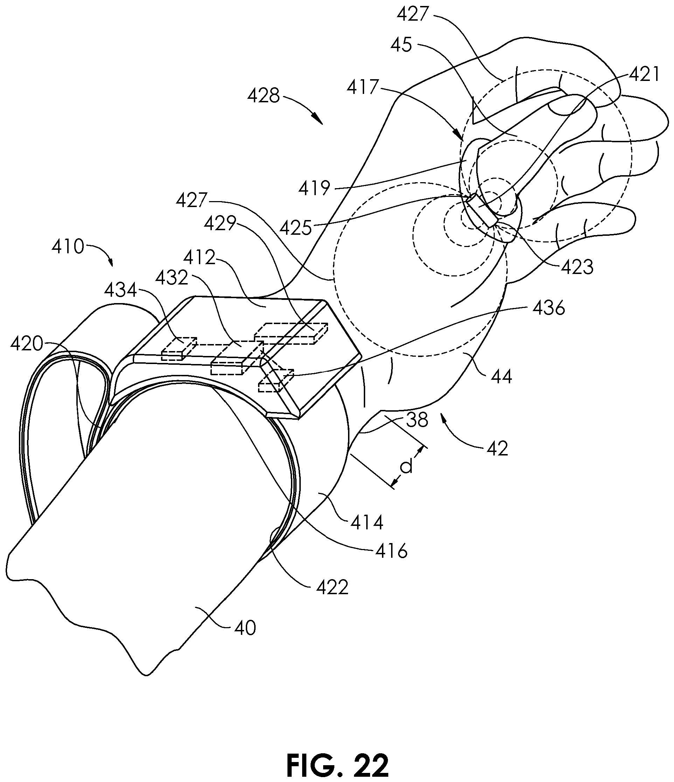

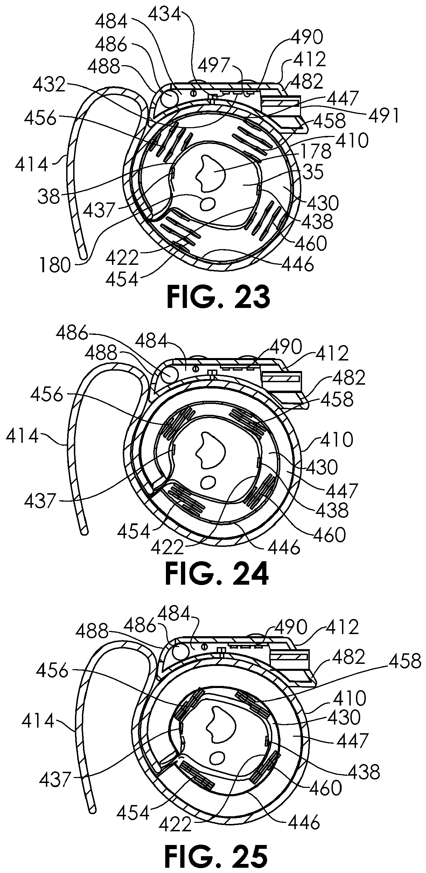

An alternative embodiment of the wearable tremor control system 100 of FIG. 8 is illustrated in FIGS. 22-27. In FIG. 22, a wearable tremor control system 410 is shown in use, in place on the wrist 38 of the arm 40 of a user 42. A band 414 of the wearable tremor control system 410 may be secured immediately adjacent the hand 44 of the user 42, or may be attached around the wrist 38 (or other portion of the arm 40) a distance d away from the hand 44, for example 0.5 cm, 1 cm, 2 cm, 5 cm, 10 cm, or 15 cm, or any distance between 0 cm and 15 cm. The wearable tremor control system 410 comprises a housing 412, with the band 414 coupled to an underside 416 of the housing 412 by epoxy or adhesive. In other embodiments, the band 414 may be secured to the housing 412 by fasteners, sewing, fusing, or may slide through slits or elongate spaces in the housing 412. The band 414 is configured to wrap around the wrist 38 of the user/patient 42 and secure to itself by use of a hook and loop (Velcro®-type) system 420 or an alternative closure system. In some embodiments, the band 414 may be configured to be worn like a watch or a bracelet, and may be configured to partially or fully encircle a limb (arm, leg) at a portion (wrist, ankle, etc.). The hook and loop system 420 may be replaced in alternative embodiments by a button closure, a snap closure, an adhesive closure, or a magnetic closure. A controller 432 within the housing 412 is configured to receive signals from a sensor 428. The sensor 428 comprises a ring 417 configured to encircle a finger of the user 42. In FIG. 22 the ring 417 is in place on the thumb 45 of the hand 44. The ring 417 includes a band 419 for encircling the thumb 45 and a magnet 421 attached to the band 419. The magnet 421 is positioned on the band 419 so that its north pole 423 and south pole 425 can be oriented as shown. In this orientation of a variation thereof, a baseline magnetic field is created in proximity to the wrist 38, as illustrated by magnetic field lines 427. The sensor 428 also includes a magnetometer 429, shown disposed within the housing 412. The magnetometer 429 is configured to sense changes to the magnetic field 427 which may be caused by disturbances from tremors in the arm 40, wrist 38, or hand 44 of the patient 42. The magnetometer 429 is coupled to the controller 432, and may comprise a digital 3-axis magnetometer, such as an LIS3MDL supplied by Pololu Corporation, Las Vegas, NV. The LIS3MDL provides magnetic field strength measurements with a configurable range of ±4 gauss to ±16 gauss that can be read through a digital I²C or SPI interface. The magnet 421 may comprise a rare earth magnet, such as a neodymium-iron-boron magnet or samarium cobalt magnet, and may have a grade of N45 or greater, or N48 or greater, or N52 or greater. The baseline magnitude of the magnetic field (magnetic flux density) supplied by the magnet 421 may be around 1.0 to 3.0 Gauss, measured at or near the wrist 38. As a comparison, the magnetic flux density of the earth's magnetic field is between about 0.2 Gauss and 0.7 Gauss, thus, the magnetic field of the magnet 421 is distinct. The magnet 421 is shown in FIG. 22 as a cylindrical magnet, but other configurations may be used, including semi-cylindrical or disk. The magnetometer 429 is also configured to allow the dynamic assessment of oscillatory/frequency characteristics of the changes in the magnetic field 427. The controller 432 may be configured to recognize dynamic activity in the measured magnetic field 427 that is in the frequency range of typical tremors. The sensor 428 may be a stand-alone detector of tremors, or may be an initial detection system which can be further corroborated by other sensors such as the sensors (sensor 28 or sensing elements 132, 134) previously described. Vibration elements 437, 438 (FIGS. 23-25) may optionally be included, and may be configured to be operated by the controller 432.

The controller 432 may include a microcontroller, including any described herein. The controller 432 may be coupled to a transceiver 434, configured to communicate wirelessly to a cellular phone, smart phone, or other personal communication device, including a chip implanted in a user's body, or carried on a portion of the user's body or clothing. The transceiver 434 may comprise a wifi antenna. An actuator 436 (automated pump, jack, etc.), coupled to the controller 432 is configured to receive signals from the controller 432 to cause an inflatable inner cuff 446 (FIGS. 23-25) to expand within the interior space 430 of an outer cuff 422.

FIG. 23 illustrates the wearable tremor control system 410 in use on a wrist 38 of a user 42 with the inner cuff 446 in a first, substantially uninflated, state. Radius 178 and ulna 180 bones are shown in the cross-section of the wrist 38, as the cross-section is taken through a portion of the wearable tremor control system 410 that is proximal to the carpal bones. Muscle 35 is also shown surrounding the radius 178 and ulna 180. FIG. 24 illustrates the wearable tremor control system 410 in use on a wrist 38 of a user 42 with the inner cuff 446 in a second, semi-inflated or partially inflated, state. FIG. 25 illustrates the wearable tremor control system 410 in use on a wrist 38 of a user 42 with the inner cuff 446 in a third, substantially inflated, state. The increase in the volume of the interior space 447 of the inner cuff 446 is visible, progressing from FIG. 23 to FIG. 24 and from FIG. 24 to FIG. 25. The resultant increased compression of the compression springs 454, 456, 458, 460 is also visible.

The housing 412 of the wearable tremor control system 410 comprises a wall 482 and an internal cavity 484. A battery 486 is held within the internal cavity 484 and covered with a removable batter cover 488. The battery 486 is configured to power a circuit board 490 of the wearable tremor control system 410. The circuit board 490 includes the controller 432, which is configured to control the actuator 436 (FIG. 22). The circuit board 490 also includes the transceiver 434 which is configured to communicate with an external device 193 (FIGS. 15 and 18), such as a smart phone, pad, personal computer, or other device capable of communication. The external device 193 may include an application (App) 189 that allows the users to control and modify the operation of the wearable tremor control system 410. The housing 412 additionally includes a connection port 491 for transferring data, or transferring energy (e.g., to allow charging). The connection port 491 may comprise a USB port, USB Type-3, Thunderbolt, Thunderbolt 3, etc. Connectivity to the application 189 may be accomplished using one of several wireless technologies such as Bluetooth or wifi.

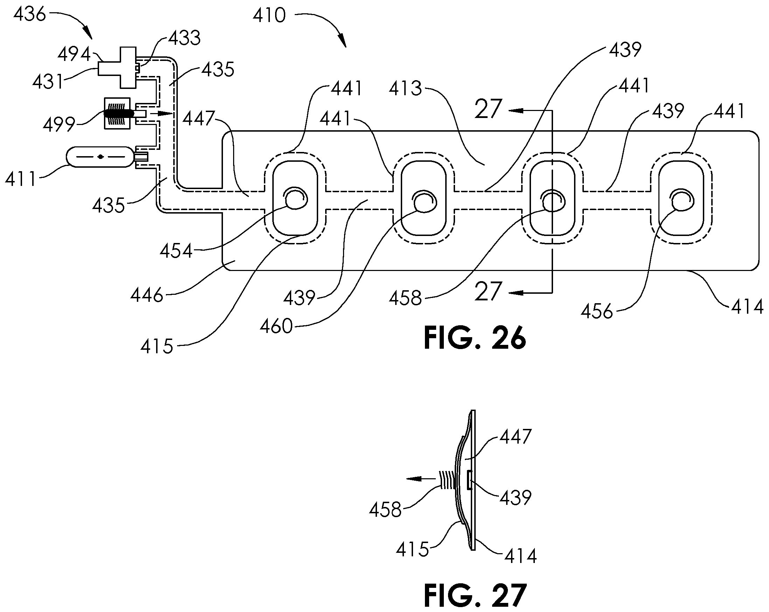

Turning to FIGS. 26 and 27, the wearable tremor control system 410 differs from the wearable tremor control system 100 of FIG. 8 because the actuator 436 and the band 414 each contain several new features. The actuator 436 includes a rolling micro-pump 494 which is configured to pump air in from an inlet 431 and out through an outlet 433 into a conduit 435. The rolling micro-pump 494 may be configured to achieve a no-load flow rate of between 0.02 liters/minute to 0.15 liters/minute, or between 0.07 liters/minute to 0.12 liters/minutes, or between about 0.08 liters/minute and about 0.10 liters/minute. In some embodiments, the rolling micro-pump 494 may comprise an Oken RSP08D01R. Alternatively, a piezo pump may be used, such as piezo pumps commonly used in non-invasive blood pressure monitors (NIBP). In some embodiments, a Takasago Fluidic Systems SDMP320/330W pump may be utilized, which has a typical flow rate of 0.02 liters/minute to 0.03 liters/minute. A piezo pump such as the SDMP320/330W has a relatively flat profile, allowing it to fit into small spaces (e.g., within the internal cavity 484 (FIG. 25). A solenoid valve 499 is may be controlled by the controller 432 (FIG. 23) to open up or close off the conduit 435, closing (arrow) to maintain pressure achieved by the pumping of the rolling micro-pump 494, and opening (position shown) to allow the release of pressure. A pressure sensor 411 is configured to send a signal (e.g., to the controller 432) based on the pressure it measures within the conduit 435. By continuously or intermittently sensing the pressure with the pressure sensor 411, the interior space 447 of the inner cuff 446 may be pressurized in a precision manner. When the pressure approaches a predetermined or pre-calculated set point, the controller 432 may switch to operating the rolling micro-pump 494 and/or solenoid valve 499 to maintain the set point pressure. The pressure sensor 411 may include analog or digital output. An exemplary sensor is a Panasonic ADP5240, having a suitable sensing range of 0 kPa to 100 kPa. Returning to FIG. 23, the circuit board 490 further comprises a memory unit 497 which is configured to store data, such as patient data, calibration data, treatment programs, treatment data (e.g., reduction or increase in amplitude, intensity and/or prevalence of tremor), and measurement algorithms.

The time to pressurize the interior space 447 of the inner cuff 446 may be minimized for efficiency sake by increasing the flow rate capacity of the pump used. Alternatively, the interior space 447 may be minimized, and thus optimized, by having reduced width or profile areas 439 between the compression springs 454, 456, 458, 460, and wide areas 441 immediately surrounding the compression springs 454, 456, 458, 460. Thus, the portion of the conduit 435 extending through the band 414 and forming the interior space 447 has a volume that is not significantly larger than needed. Non-inflatable portions 413 of the band 414 surround the reduced width areas 439. Thus, the inner cuff 446 only inflates where it needs to inflate, preferentially under the compression springs 454, 456, 458, 460. The further reinforce the compression springs 454, 456, 458, 460, a rigid base 415 is coupled to the inner cuff 446 on the side adjacent to the outer cuff 422, to which the compression springs 454, 456, 458, 460 may be coupled. This is shown in more detail in FIG. 27. In some embodiments, the rigid base 415 may comprise a polyimide (e.g., Kapton®) sheet. Furthermore, a major component of the force applied by the compression spring 454, 456, 458, 460 is proportional to the rigid base 415 area which couples the compression springs 454, 456, 458, 460 to the inner cuff 446. Thus, coupling to the wrist 38 (or other anatomical feature) of the patient 42 is increased. The same type of focused coupling using a rigid base 415 may be used with vibration elements 437, 438 (FIG. 23). A vibration element 437, 438 that is forced against the wrist 38 of the patient 42 with increased pressure can be more effective, as can a compression spring 454, 456, 458, 460. In some embodiments, the rigid base 415 may also serve as an acoustic coupling for the vibration elements 437, 438 to the outer cuff 422, for example, having a matched acoustic impedance. The coupling may be to an outer wall surface (as in FIGS. 23-25) or alternatively an inner wall surface of the outer cuff 422. An alternative manner of increasing the pressure at which the vibration elements 437, 438 are applied is to place them preferentially under the portion of the outer cuff 422 that is directly under the housing 412, which itself tends to be more rigid, thus allowing for increase push or pre-load of the vibration elements 437, 438 against the wrist 38. The embodiments described thus provide potential for a medication-free tremor management option for patients. The discreet nature of the device, which appears similar to a traditional watch or smartwatch, allows a user to minimize attention or social embarrassment.

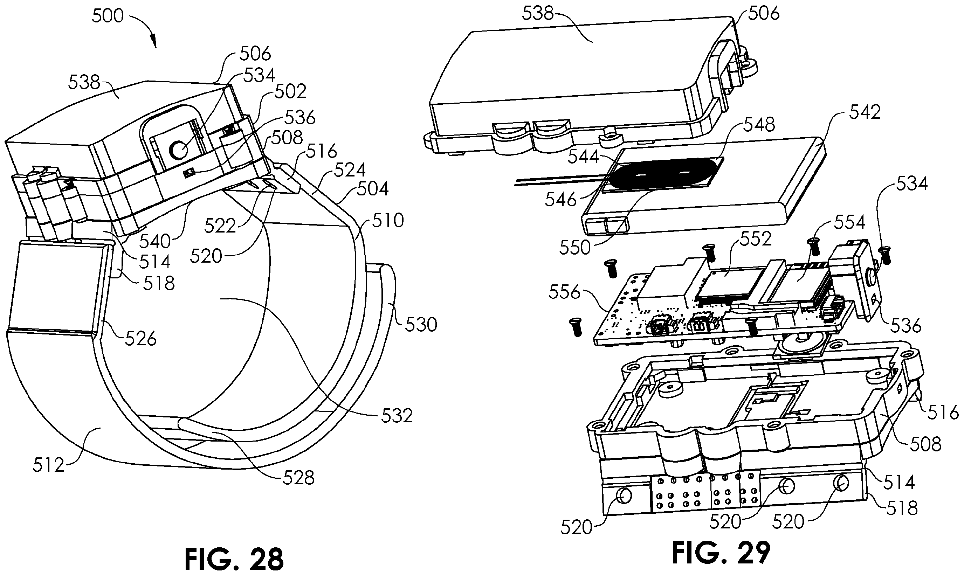

FIGS. 28-30 illustrate a wearable tremor control system 500 having a housing 502 and a band 504. The housing 502 comprises a housing top 506 and a housing bottom 508 which are configured to be attached to each other, as shown in FIG. 28. The band 504 comprises a first band portion 510 and a second band portion 512. The housing bottom 508 is coupled to a base 514 having a first bracket 516 and second bracket 518, the brackets having holes 520. Screws 522 connect the first bracket 516 to the first band portion 510 at its first end 524 and connect the second bracket 518 to the second band portion 512 at its first end 526. The second end 528 of the first band portion 510 and the second end 530 of the second band portion 512 overlap each other. In some embodiments, a hook-and-loop (e.g., Velcro®) connection may exist between the first band portion 510 and the second band portion 512. In other embodiments, the first band portion 510 and the second band portion 512 may each be made of a material such as a high durometer elastomer, having sufficient thickness, so that the first band portion 510 and the second band portion 512 may be flexed out of the way while the user places the wrist or other limb portion into the central opening 532, but have sufficient stiffness to maintain the band 504 in place on the wrist or other limb portion.

An on/off button 534 is located on the housing 502 for easy access by a user, and an LED 536 or other indicator demonstrates whether the wearable tremor control system 500 is operation or shut off, or in standby mode. Multiple color LEDs may be used to indicate status (for example, green for on, orange or yellow for standby, red for operational error). Though not shown in FIG. 28, a display or touch screen may be carried on an upper face 538 of the housing top 506, as described in relation to the prior embodiments. Any combination of sensors (piezoelectric crystals, accelerometers, gyroscopes, EMC sensors), stimulation electrodes, or vibration elements (e.g., piezoelectric crystals) may be carried on the band 504, on the underside 540 of the base 514, or in the housing 502.

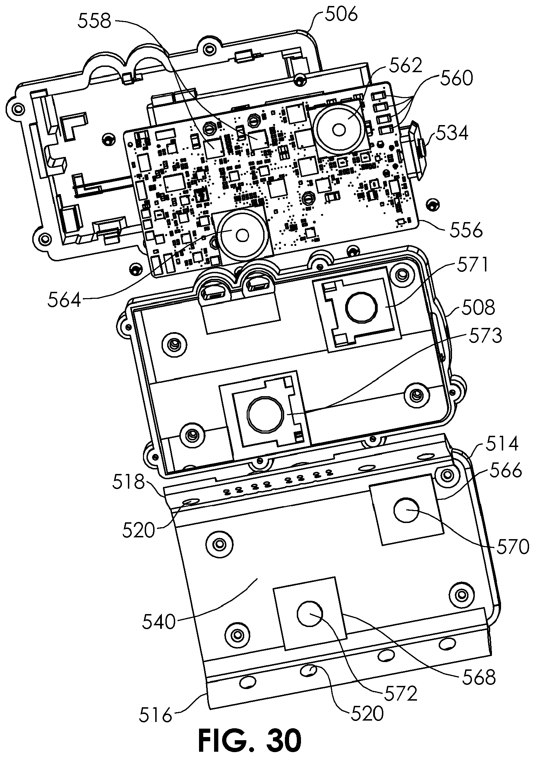

Turning to FIG. 29, within the housing 502 is a wirelessly chargeable battery 542, which may be wirelessly charged via a wireless power charging coil 544, which comprises a flat coil 546, a ferrite sheet 548, and a dielectric sheet 550 and EMI shield. The wireless power charging coil 544 receives current from wireless charging units via inductive coupling, to charge the battery 542. Thin wireless power charging coils 544 can be obtained from Wurth Electronik eiSos GmbH & Co of Waldenburg, Germany. A microcontroller 552 and a transceiver 554 are carried on a circuit board 556, and function as described in relation to the microcontrollers and transceivers of the prior embodiments. Other portions of the circuit board are visible in FIG. 30, such as an AC/DC converter 558, which may comprise rectifiers, and a transient voltage suppression unit 560, which may comprise voltage-dependent resistors (varistors).

Piezo haptic actuators 562, 564 are carried within the housing 502 and are configured to actuate (displace) when a voltage is applied on them. The base 514 includes openings 566, 568 and the housing bottom 508 includes openings 571, 573 over which membranes 570, 572 are placed, respectively. Openings 566, 571 are aligned with membrane 570 and openings 568, 573 are aligned with membrane 572. The displacement of the piezo haptic actuators 562, 564 displace the membranes 570, 572, respectively, such that when the wearable tremor control system 500 is in place on a user's wrist, the movement and force applied directly on the user's skin by the membranes 570, 572 provide haptic feedback. The haptic feedback may be initiated to warn the user of events such as device powering on, device powering off, device error, treatment starting, treatment ending, measurement starting, measurement ending, data being generated, treatment plan being changed, request or suggestion to contact physician or medical care, or other commands. Additionally, or alternatively, the haptic feedback may be used to provide treatment to the patient via direct pressure on portions of the user's wrist over which the membranes 570, 572, and thus actuators 562, 564, are located. The applied pressure is somewhat analogous to the compression applied by an inflatable or expandable cuff described in earlier embodiments. In some embodiments, the piezo haptic actuator may comprise a PowerHap™ 7G supplied by EPCOS AG of Munich, Germany.

An energy modulation algorithm may be applied, allowing any of the wearable tremor control systems 10, 100, 210, 250, 300, 410, 500 to learn and better deliver custom neuromodulation management to each wearer, which may correspond to each patient's particular tremor symptoms. Thus, an individualized treatment plan may be constructed or adapted for each patient/user. For example, the control unit (e.g., controller, microcontroller) may be configured or configurable to reduce the power output by an energy applicator (electrode, vibratory element, compression element, or other) when a signal output by a sensor (any of the sensors described herein) changes by a particular amount or by a particular value. For example, the signal output by the sensor may decrease after energy is applied by the energy applicator. Thus, the control unit is able to judge that treatment has been effective to a level that warrants the reduction of applied energy of treatment, or the reduction of duration of treatment cycles, or the cessation of treatment altogether (at least temporarily). For example, in some embodiments, the control unit may be configured or configurable to reduce the level of power which is output by the energy applicator when a signal output by a sensor following an application of energy by the energy applicator is less than about 80 percent of the signal output by the sensor prior to the application of energy by the energy applicator. When two types of energy are being used (e.g., electrical stimulation and vibration), the reduction in power can be a reduction of only one of the two types of energy, or a reduction of both types of energy. In some embodiments, the control unit may be configured or configurable to reduce the level of power which is output by the energy applicator when a signal output by a sensor following an application of energy by the energy applicator is less than about 50 percent of the signal output by the sensor prior to the application of energy by the energy applicator. In some embodiments, the control unit may be configured or configurable to reduce the level of power which is output by the energy applicator when a signal output by a sensor following an application of energy by the energy applicator is less than about 20 percent of the signal output by the sensor prior to the application of energy by the energy applicator. In some embodiments, the control unit may be configured or configurable to reduce the level of power which is output by the energy applicator when a signal output by a sensor following an application of energy by the energy applicator is less than about 10 percent of the signal output by the sensor prior to the application of energy by the energy applicator.

In some embodiments, the control unit can be configured or configurable to change an output parameter of electrical stimulation and an output of vibration independently of each other. The output parameter of electrical stimulation to be changed may include voltage, current, power, frequency, duration, or amplitude. The output parameter of vibration to be changed may include power, frequency, harmonic mode number, duration, or amplitude. In some embodiments, the control unit can be configured to control the operation of the energy applicator based at least upon a patient activity characteristic. Examples of patient activity characteristics that may be used are: eating, drinking, walking, running, sleeping, resting while awake, sitting, talking, meditating, typing, or writing. A memory unit in the wearable tremor control system 10, 100, 210, 250, 300, 410, 500 may be used to store one of more of the patient activity characteristics, for example, for later use. In some embodiments, a multi-modal energy applicator may also be used as a multi-modal sensor. For example, combination of one or more electrodes for electrical stimulation and one or more piezo elements for therapeutic vibration may also have the capability to sense stimuli and activity of muscles during active tremors. The control unit may also be able to switch between "sense" modes, wherein signals from the electrode(s) and piezo element(s) are received and processed and "delivery" modes, wherein the electrode(s) and piezo element(s) are purposely excited for the purpose of delivering energy. The control unit may also provide a feedback loop, wherein the amount of activity in the "delivery" mode is dependent upon the measured signals in the "sense" mode. In any of the embodiments described, the data may be shared wirelessly with others, including medical personnel. The particular algorithm of the feedback loop may be manually adjusted by the user or others using the user interface, or manually adjusted by medical personnel or others in a wireless manner. Alternatively, the particular algorithm of the feedback loop may automatically adjust, depending on changes in particular parameters.

Any of the embodiments described above may be configured to be used on the arms, hands, legs, or feet such that one or more the electrodes 252, 254, 256 or 302, 304, 306 is capable of treating one or more of the nerves of the arms, hands, legs, or feet that are in communication with the central nervous system. The nerves in particular are those that may be responsible for tremors or involuntary movements. The nerves may include, but are not limited to median nerves. Other nerves that are a target for treatment by apparatus of the embodiments presented herein include the radial nerve and the ulnar nerve.

In some embodiments, including variations of the embodiments disclosed above, the user interface 101, 312 may be configured to remotely communicate with the electronics of the housing 12, 102, 336, 412, 502 for example via Bluetooth, wifi, or other wireless networks. The user interface 101, 312 may also be configured to be attachable, on its own, to portions of a user's body, such as a wrist, arm, ankle, leg, head, waist, or neck, for example by having a belt, an elastic band or a band that can be tied. In still other embodiments, there may be a wired connection, such as an extensible wire, between the user interface 101, 312 and the housing 12, 102, 336, 412, 502.

In some embodiments, one or more of the piezoelectric crystals described herein may be mounted to the bands 14, 104, 310, 414, 504 via a rigid or semi-rigid backing, such as polyimide (Kapton). Furthermore, the piezoelectric crystals may include a mechanical displacement amplifier to improve energy transfer to a wearer/patient. The mechanical displacement amplifier may include a resonator or an oscillator.

In one embodiment of the present disclosure, a system for treatment of involuntary muscle contraction comprises a wearable interface having an internal contact surface, the wearable interface configured to at least partially encircle a first portion of a limb of a subject, a sensory module including a magnetic element configured to be worn in proximity to the first portion of the limb of the subject and a magnetic sensor, and an energy application module carried by the wearable interface and configured to apply one or more forms of mechanical energy to the limb, wherein the energy application module is capable of changing the character of the one of more forms of mechanical energy. In some embodiments, the energy application module is configured to change the character of the one or more forms of mechanical energy in response to changes in the signal output from the sensory module. In some embodiments, the magnetic element comprises a magnet. In some embodiments, the magnet comprises a rare earth magnet, such as neodymium-iron-boron, or samarium-cobalt. In some embodiments, the magnetic sensor comprises a magnetometer. In some embodiments, the energy application module comprises one or more compression springs. In some embodiments, the energy application module comprises one or more piezoelectric elements. In some embodiments, the energy application module is coupled to a cuff via one or more rigid sheets. In some embodiments, the cuff is an inflatable cuff.

While the foregoing is directed to embodiments of the present invention, other and further embodiments of the invention may be devised without departing from the basic scope thereof.

The ranges disclosed herein also encompass any and all overlap, sub-ranges, and combinations thereof. Language such as "up to," "at least," "greater than," "less than," "between," and the like includes the number recited. Numbers preceded by a term such as "approximately", "about", and "substantially" as used herein include the recited numbers (e.g., about 10%=10%), and also represent an amount close to the stated amount that still performs a desired function or achieves a desired result. For example, the terms "approximately", "about", and "substantially" may refer to an amount that is within less than 10% of, within less than 5% of, within less than 1% of, within less than 0.1% of, and within less than 0.01% of the stated amount.

## Claims

1. A system for treatment of involuntary muscle contraction, comprising:
a wearable interface (104, 310, 414, 504) having an internal contact surface (140, 308), the wearable interface (104, 310, 414, 504) configured to at least partially encircle a first portion of a limb of a subject; and
an energy applicator carried by the wearable interface (104, 310, 414, 504) and configured to apply vibrational energy and electrical stimulation energy to the limb of the subject, **characterised in that** the energy applicator is configured to apply the vibrational energy at a frequency of between about 15 kHz and about 1 MHz.

2. The system of claim 1, wherein the vibrational energy is provided by one or more piezoelectric elements (136, 138, 326, 328) carried by the wearable interface (104, 310, 414, 504).

3. The system of claim 2, wherein at least one of the one or more piezoelectric elements (136, 138, 326, 328) is configured to vibrate at a frequency of between about 20 kHz and about 700 kHz.

4. The system of claim 2, wherein the one or more piezoelectric elements (136, 138, 326, 328) comprise a first piezoelectric element configured to vibrate at a first frequency and a second piezoelectric element configured to vibrate at a second frequency, different from the first frequency.

5. The system of claim 4, wherein the first frequency is between about 1 Hz and about 30 Hz and the second frequency is between about 20 kHz and about 1 MHz.

6. The system of claim 1, wherein the electrical stimulation energy is provided by one or more electrodes (252, 254, 256, 302, 304, 306) carried by the wearable interface (104, 310, 414, 504).

7. The system of claim 1, further comprising a control unit (192, 314, 432) configured to control the operation of the energy applicator.

8. The system of claim 7, wherein the control unit (192, 314, 432) is programmable.

9. The system of claim 7, wherein the control unit (192, 314, 432) is configured to modify the operation of the energy applicator over time.

10. The system of claim 7, further comprising a sensor (132, 134, 316, 318) carried by the wearable user interface (104, 310, 414, 504) and configured to output a signal related to muscular contraction within the limb.

11. The system of claim 10, wherein the control unit (192, 314, 432) is configured to modify the operation of the energy applicator based at least in part on measured changes in the signal output by the sensor (132, 134, 316, 318).

12. The system of claim 11, wherein the control unit (192, 314, 432) is configured to change at least one of an amplitude or a frequency of energy applied by the energy applicator in response to a change in an amplitude of the signal output by the sensor (132, 134, 316, 318).

13. The system of claim 11, wherein the control unit (192, 314, 432) is configured to increase at least one of an amplitude or a frequency of energy applied by the energy applicator in response to an increase in an amplitude of the signal output by the sensor (132, 134, 316, 318).

14. The system of any one of claims 11-13, wherein the control unit (192, 314, 432) is configured to modify the operation of the energy applicator in a manner that is proportional to the amplitude, intensity and/or prevalence of tremors in the limb of the subj ect.

15. The system of any one of claims 10-14, further comprising a memory configured to store one or more patient activity characteristics.

16. The system of claim 15, wherein the one or more patient activity characteristics comprise one or more of: eating, drinking, walking, running, sleeping, resting while awake, sitting, talking, meditating, typing, or writing.

17. The system of any one of claims 10-16, wherein the electrical stimulation energy is provided by one or more electrodes (252, 254, 256, 302, 304, 306) carried by the wearable interface (104, 310, 414, 504), and wherein the control unit (192, 314, 432) is configured to increase or decrease one or more parameters of the one or more electrodes (252, 254, 256, 302, 304, 306).

18. The system of claim 17, wherein the one or more parameters include at least a parameter selected from the list consisting of: voltage, current, frequency, or pulse width,

19. The system of either one of claims 17 or 18, wherein the control unit (192, 314, 432) is configured to activate the one or more electrodes (252, 254, 256, 302, 304, 306) in one or more pattern selected from the list consisting of: a biphasic sine wave, a multiphasic wave, a monophasic sine wave, a biphasic pulsatile sine wave, a biphasic rectangular wave, a monophasic square wave, a monophasic pulsatile rectangular wave, a biphasic spiked wave, a monophasic spiked wave, and a monophasic pulsatile spiked wave.

20. The system of any one of claims 7-19, wherein the control unit (192, 314, 432) is configured to provide an individualized treatment plan constructed or adapted for the subj ect.

21. The system of claim 20, wherein the control unit (192, 314, 432) is configured to automatically construct or adapt the individualized treatment plan.

22. The system of any one of claims 1-21, wherein the wearable interface (104, 310, 414, 504) comprises a wristband configured to at least partially encircle a wrist of the subj ect.

23. The system of claim 22, wherein the energy applicator is carried adjacent the internal contact surface (140, 308) and configured to contact the skin of the wrist of the subj ect.

24. The system of any one of claims 1-9, wherein the energy applicator is configured to create a mixed signal including a particular combination of vibration and electrical stimulation.

25. The system of claim 24, wherein the mixed signal includes a first independent period comprising only one of vibration or electrical stimulation and a second independent period comprising only the other of vibration or electrical stimulation.

26. The system of either one of claims 24 or 25, wherein the mixed signal includes a first combined period comprising both vibration and electrical stimulation and a second combined period comprising both vibration and electrical stimulation, wherein the first combined period has a first ratio between the amount of vibration and the amount of electrical stimulation and the second combined period has a second ratio between the amount of vibration and the amount of electrical stimulation, the second ratio different from the first ratio.

## Patentansprüche

1. System zur Behandlung unwillkürlicher Muskelkontraktionen, umfassend:
eine anlegbare Schnittstelle (104, 310, 414, 504) mit einer inneren Kontaktfläche (140, 308), wobei die anlegbare Schnittstelle (104, 310, 414, 504) so gestaltet ist, dass sie einen ersten Abschnitt einer Gliedmaße eines Patienten zumindest zum Teil umgibt; und
einen Energieapplikator, der von der anlegbaren Schnittstelle (104, 310, 414, 504) getragen wird und dafür ausgelegt ist, Schwingungsenergie und Stromimpulsenergie auf die Gliedmaße des Patienten aufzubringen,
**dadurch gekennzeichnet, dass**
der Energieapplikator so gestaltet ist, dass er die Schwingungsenergie mit einer Frequenz zwischen etwa 15 kHz und etwa 1 MHz aufbringt.

2. System nach Anspruch 1, wobei die Schwingungsenergie von einem oder mehreren piezoelektrischen Elementen (136, 138, 326, 328), die auf der anlegbaren Schnittstelle (104, 310, 414, 504) getragen werden, bereitgestellt wird.

3. System nach Anspruch 2, wobei das eine oder mindestens eines von den mehreren piezoelektrischen Elementen (136, 138, 326, 328) so gestaltet ist, dass es mit einer Frequenz zwischen etwa 20 kHz und etwa 700 kHz schwingt.

4. System nach Anspruch 2, wobei das eine oder die mehreren piezoelektrischen Elemente (136, 138, 326, 328) umfasst: ein erstes piezoelektrisches Element, das so gestaltet ist, dass es mit einer ersten Frequenz schwingt, und ein zweites piezoelektrisches Element, das so gestaltet ist, dass es mit einer zweiten, von der ersten Frequenz verschiedenen Frequenz schwingt.

5. System nach Anspruch 4, wobei die erste Frequenz zwischen etwa 1 Hz und etwa 30 Hz liegt und die zweite Frequenz zwischen etwa 20 kHz und etwa 1 MHz liegt.

6. System nach Anspruch 1, wobei die elektrische Stimulationsenergie von einer oder mehreren Elektroden (252, 254, 256, 302, 304, 306) bereitgestellt wird, die auf der anzulegenden Schnittstelle (104, 310, 414, 504) getragen werden.

7. System nach Anspruch 1, ferner eine Steuereinheit (192, 314, 432) umfassend, die so gestaltet ist, dass sie den Betrieb des Energieapplikators steuert.

8. System nach Anspruch 7, wobei die Steuereinheit (192, 314, 432) programmierbar ist.

9. System nach Anspruch 7, wobei die Steuereinheit (192, 314, 432) so gestaltet ist, dass sie den Betrieb des Energieapplikators im Zeitverlauf modifiziert.

10. System nach Anspruch 7, ferner einen Sensor (132, 134, 316, 318) umfassend, der von der anlegbaren Schnittstelle (104, 310, 414, 504) getragen wird und dafür ausgelegt ist, ein Signal auszugeben, das eine Muskelkontraktion innerhalb der Gliedmaße betrifft.

11. System nach Anspruch 10, wobei die Steuereinheit (192, 314, 432) so gestaltet ist, dass sie den Betrieb des Energieapplikators zumindest zum Teil auf Basis von gemessenen Änderungen an dem Signal, das von dem Sensor (132, 134, 316, 318) ausgegeben wird, modifiziert.

12. System nach Anspruch 11, wobei die Steuereinheit (192, 314, 432) so gestaltet ist, dass sie als Reaktion auf eine Änderung an einer Amplitude des von dem Sensor (132, 134, 316, 318) ausgegebenen Signals eine Amplitude und/oder eine Frequenz einer Energie, die von dem Energieapplikator aufgebracht wird, zu ändern.

13. System nach Anspruch 11, wobei die Steuereinheit (192, 314, 432) so gestaltet ist, dass sie als Reaktion auf eine Erhöhung einer Amplitude des von dem Sensor (132, 134, 316, 318) ausgegebenen Signals eine Amplitude und/oder eine Frequenz einer Energie, die von dem Energieapplikator aufgebracht wird, erhöht.

14. System nach einem der Ansprüche 11-13, wobei die Steuereinheit (192, 314, 432) so ausgelegt ist, dass sie den Betrieb des Energieapplikators auf eine Weise modifiziert, die proportional ist zu der Amplitude, der Intensität und/oder zu einer Prävalenz eines Zitterns in der Gliedmaße des Patienten.

15. System nach einem der Ansprüche 10-14, ferner einen Speicher umfassend, der so gestaltet ist, dass er einen oder mehrere Patientenaktivitätskennwerte speichert.

16. System nach Anspruch 15, wobei der eine oder die mehreren Patientenaktivitätskennwerte eines oder mehrere umfassen von: Essen, Trinken, Gehen, Laufen, Schlafen, Ruhen im Wachzustand, Sitzen, Sprechen, Meditieren, Schreiben mittels einer Tastatur oder Schreiben mit einem Stift.

17. System nach einem der Ansprüche 10-16, wobei die elektrische Stimulationsenergie von einer oder mehreren Elektroden (252, 254, 256, 302, 304, 306) bereitgestellt wird, die auf der anzulegenden Schnittstelle (104, 310, 414, 504) getragen werden, und wobei die Steuereinheit (192, 314, 432) so gestaltet ist, dass sie einen oder mehrere Parameter der einen oder der mehreren Elektroden (252, 254, 256, 302, 304, 306) erhöht oder senkt.

18. System nach Anspruch 17, wobei der eine oder die mehreren Parameter zumindest einen Parameter einschließen, der ausgewählt ist aus der Liste bestehend aus: Spannung, Strom, Frequenz oder Impulsbreite.

19. System nach einem der Ansprüche 17 oder 18, wobei die Steuereinheit (192, 314, 432) so gestaltet ist, dass sie eine oder mehrere Elektroden (252, 254, 256, 302, 304, 306) in einem oder in mehreren Mustern aktiviert, die ausgewählt sind aus der Liste bestehend aus: einer zweiphasigen Sinuswelle, einer mehrphasigen Welle, eine einphasigen Sinuswelle, einer zweiphasigen gepulsten Sinuswelle, einer zweiphasigen Rechteckwelle, einer einphasigen quadratischen Welle, einer einphasigen gepulsten Rechteckwelle, einer zweiphasigen Spike-Welle und einer einphasigen gepulsten Spike-Welle.

20. System nach einem der Ansprüche 7-19, wobei die Steuereinheit (192, 314, 432) so gestaltet ist, dass sie einen individualisierten Behandlungsplan bereitstellt, der für den Behandelten erstellt oder angepasst ist.

21. System nach Anspruch 20, wobei die Steuereinheit (192, 314, 432) so gestaltet ist, dass sie den individualisierten Behandlungsplan automatisch erstellt oder anpasst.

22. System nach einem der Ansprüche 1-21, wobei die anlegbare Schnittstelle (104, 310, 414, 504) ein Armband umfasst, das so gestaltet ist, dass sie ein Handgelenk des Behandelten zumindest zum Teil umgibt.

23. System nach Anspruch 22, wobei der Energieapplikator angrenzend an die innere Kontaktfläche (140, 308) getragen wird und so gestaltet ist, dass sie die Haut des Handgelenks des Behandelten berührt.

24. System nach einem der Ansprüche 1-9, wobei der Energieapplikator so gestaltet ist, dass er ein Mischsignal erzeugt, das eine bestimmte Kombination aus Schwingung und elektrischer Stimulation einschließt.

25. System nach Anspruch 24, wobei das Mischsignal eine erste unabhängige Periode einschließt, die nur entweder eine Schwingung oder eine elektrische Stimulation umfasst, und eine zweite unabhängige Periode, die nur das jeweils andere von der Schwingung oder der elektrischen Stimulation umfasst.

26. System nach einem der Ansprüche 24 oder 25, wobei das Mischsignal einschließt: eine erste kombinierte Periode, die sowohl Schwingung als auch elektrische Stimulation umfasst, und eine zweite kombinierte Periode, die sowohl Schwingung als auch elektrische Stimulation umfasst, wobei die erste kombinierte Periode ein erstes Verhältnis zwischen dem Maß der Schwingung und dem Maß der elektrischen Stimulation aufweist und die zweite kombinierte Periode ein zweites Verhältnis zwischen dem Maß der Schwingung und dem Maß der elektrischen Stimulation, das von dem ersten Verhältnis verschieden ist, aufweist.

## Revendications

1. Système pour le traitement de la contraction musculaire involontaire, comprenant :
une interface portable (104, 310, 414, 504) ayant une surface de contact interne (140, 308), l'interface portable (104, 310, 414, 504) étant configurée pour encercler au moins partiellement une première partie d'un membre d'un sujet ; et
un applicateur d'énergie porté par l'interface portable (104, 310, 414, 504) et étant configuré pour appliquer une énergie vibratoire et une énergie de stimulation électrique au membre du sujet, **caractérisé en ce que** l'applicateur d'énergie est configuré pour appliquer l'énergie vibratoire à une fréquence comprise entre environ 15kHz et environ 1 MHz.

2. Système selon la revendication 1, dans lequel l'énergie vibratoire est fournie par un ou plusieurs éléments piézoélectriques (136, 138, 326, 328) portés par l'interface portable (104, 310, 414, 504).

3. Système selon la revendication 2, dans lequel au moins l'un de l'un ou des plusieurs éléments piézoélectriques (136, 138, 326, 328) est configuré pour vibrer à une fréquence comprise entre environ 20 kHz et environ 700 kHz.

4. Système selon la revendication 2, dans lequel le ou les éléments piézoélectriques (136, 138, 326, 328) comprennent un premier élément piézoélectrique configuré pour vibrer à une première fréquence et un deuxième élément piézoélectrique configuré pour vibrer à une deuxième fréquence, différente de la première fréquence.

5. Système selon la revendication 4, dans lequel la première fréquence est comprise entre environ 1 Hz et environ 30 Hz et la deuxième fréquence est comprise entre environ 20 kHz et environ 1 MHz.

6. Système selon la revendication 1, dans lequel l'énergie de stimulation électrique est fournie par une ou plusieurs électrodes (252, 254, 256, 302, 304, 306) portées par l'interface portable (104, 310, 414, 504).

7. Système selon la revendication 1, comprenant en outre une unité de commande (192, 314, 432) configurée pour commander le fonctionnement de l'applicateur d'énergie.

8. Système selon la revendication 7, dans lequel l'unité de commande (192, 314, 432) est programmable.

9. Système selon la revendication 7, dans lequel l'unité de commande (192, 314, 432) est configurée pour modifier le fonctionnement de l'applicateur d'énergie au fil du temps.

10. Système selon la revendication 7, comprenant en outre un capteur (132, 134, 316, 318) porté par l'interface portable d'un utilisateur (104, 310, 414, 504) et étant configuré pour émettre un signal lié à la contraction musculaire au sein du membre.

11. Système selon la revendication 10, dans lequel l'unité de commande (192, 314, 432) est configurée pour modifier le fonctionnement de l'applicateur d'énergie sur la base, au moins en partie, de changements mesurés dans le signal émis par le capteur (132, 134, 316, 318).

12. Système selon la revendication 11, dans lequel l'unité de commande (192, 314, 432) est configurée pour modifier au moins l'une parmi une amplitude et une fréquence de l'énergie appliquée par l'applicateur d'énergie en réponse à une modification de l'amplitude du signal émis par le capteur (132, 134, 316, 318).

13. Système selon la revendication 11, dans lequel l'unité de commande (192, 314, 432) est configurée pour augmenter au moins l'une parmi une amplitude et une fréquence de l'énergie appliquée par l'applicateur d'énergie en réponse à une augmentation d'une amplitude du signal émis par le capteur (132, 134, 316, 318).

14. Système selon l'une quelconque des revendications 11 à 13, dans lequel l'unité de commande (192, 314, 432) est configurée pour modifier le fonctionnement de l'applicateur d'énergie selon une façon qui est proportionnelle à l'amplitude, l'intensité et/ou la prévalence de tremblements dans le membre du sujet.

15. Système selon l'une quelconque des revendications 10 à 14, comprenant en outre une unité de mémoire configurée pour stocker une ou plusieurs caractéristiques d'activité du patient.

16. Système selon la revendication 15, dans lequel la ou les caractéristiques d'activité du patient comprennent l'un ou les actions parmi : manger, boire, marcher, courir, dormir, se reposer en étant éveillé, s'asseoir, parler, méditer, dactylographier ou écrire.

17. Système selon l'une quelconque des revendications 10 à 16, dans lequel l'énergie de stimulation électrique est fournie par une ou plusieurs électrodes (252, 254, 256, 302, 304, 306) portées par l'interface portable (104, 310, 414, 504), et dans lequel l'unité de commande (192, 314, 432) est configurée pour augmenter ou diminuer un ou plusieurs paramètres d'une ou plusieurs électrodes (252, 254, 256, 302, 304, 306).

18. Système de la revendication 17, dans lequel l'un ou les plusieurs paramètres comprennent au moins un paramètre sélectionné dans la liste constituée par la tension, le courant, la fréquence et la largeur d'impulsion.

19. Système selon l'une des revendications 17 et 18, dans lequel l'unité de commande (192, 314, 432) est configurée pour activer l'une ou les plusieurs électrodes (252, 254, 256, 302, 304, 306) en un ou plusieurs des modèles sélectionnés dans la liste constituée par : une onde sinusoïdale biphasique, une onde multiphasique, une onde sinusoïdale monophasique, une onde sinusoïdale pulsatile biphasique, une onde rectangulaire biphasique, une onde carrée monophasique, une onde rectangulaire pulsatile monophasique, une onde en pointe biphasique, une onde en pointe monophasique, et une onde en pointe pulsatile monophasique.

20. Système selon l'une quelconque des revendications 7 à 19, dans lequel l'unité de commande (192, 314, 432) est configurée pour fournir un plan de traitement individualisé construit ou adapté pour le sujet.

21. Système selon la revendication 20, dans lequel l'unité de commande (192, 314, 432) est configurée pour automatiquement construire ou adapter le plan de traitement individualisé.

22. Système selon l'une quelconque des revendications 1 à 21, dans lequel l'interface portable (104, 310, 414, 504) comprend un bracelet configuré pour au moins partiellement encercler un poignet du sujet.

23. Système de la revendication 22, dans lequel l'applicateur d'énergie est porté de façon adjacente à la surface de contact interne (140, 308) et est configuré pour faire contact avec la peau du poignet du sujet.

24. Système selon l'une quelconque des revendications 1 à 9, dans lequel l'applicateur d'énergie est configuré pour créer un signal mixte comprenant une combinaison particulière de vibrations et de stimulation électrique.

25. Système selon la revendication 24, dans lequel le signal mixte comprend une première période indépendante comprenant seulement l'une parmi les vibrations et la stimulation électrique et une deuxième période indépendante comprenant seulement l'autre parmi les vibrations et la stimulation électrique.

26. Système selon l'une des revendications 24 et 25, dans lequel le signal mixte comprend une première période combinée comprenant à la fois les vibrations et la stimulation électrique et une deuxième période combinée comprenant à la fois les vibrations et la stimulation électrique, dans lequel la première période combinée présente un premier rapport entre la quantité de vibrations et la quantité de stimulation électrique et la deuxième période combinée présente un deuxième rapport entre la quantité de vibrations et la quantité de stimulation électrique, le deuxième rapport étant différent du premier rapport.
